# EUROPEAN PATENT SPECIFICATION

(11) **EP 1 837 880 B1**
(45) Date of publication and mention of the grant of the patent: **26.08.2009**
(21) Application number: 06113480.5
(22) Date of filing: 04.05.2006
(51) Int. Cl.: G11C 8/08, G11C 16/12

(54) **Row selector for a semiconductor memory device built from low voltage transistors**
Zeilenselektor für einen Halbleiterspeicher mit Niedrigspannungstransistoren
Sélecteur de rangées pour mémoire sémiconductrice composé de transistors à basse tension

(30) Priority: 21.03.2006 EP 06111477
(43) Date of publication of application: 26.09.2007
(73) Proprietor: STMicroelectronics S.r.l., 20041 Agrate Brianza (Milano) (IT)
(72) Inventor: Campardo, Giovanni, 24128 Bergamo (BG) (IT); Micheloni, Rino, 22078 Turate (CO) (IT)
(74) Representative: W.P. Thompson & Co.

(56) References cited:
- US-A- 4 835 423
- US-A- 5 287 536
- US-A- 5 796 656
- US-A1- 2004 017 722

## Description

### Field of the invention

The present invention relates to the field of the semiconductor memory device. More specifically, the present invention relates to row selectors.

### Background art

Published United States patent application US 2004/0017722 (Cavaleri et al) was cited as the closest prior art by the European Patent Office during prosecution of this application and describes a FLASH memory whose word line decoder includes a unit for applying, when a page is being erased, a negative erase voltage to the gates of the transistors of the page to be erased, while applying a positive inhibit voltage to the gates of the transistors of at least one page that is not to be erased.

Semiconductor memory devices are commonly used to store information (either temporarily or permanently) in a number of applications; particularly, in a non-volatile memory device the information is preserved even when a power supply is off. Typically, the memory device includes a matrix of memory cells that are arranged in a plurality of rows (connected to corresponding word lines) and in a plurality of columns (connected to corresponding bit lines).

For example, flash memory devices are a particular type of non-volatile memory device, in which each memory cell is formed by a floating gate MOSFET transistor. Each memory cell has a threshold voltage (depending on the electric charge stored in the corresponding floating gate), which can be programmed to different levels representing corresponding logical values. Particularly, in a multilevel flash memory device each memory cell's threshold voltage can take more than two levels (and then store a plurality of bits).

In order to retrieve and/or store information, the flash memory device includes a decoding system that is adapted to decode an addressing code identifying a group of memory cells. In particular, the decoding system includes a row selector for selecting a corresponding word line and a column selector for selecting a corresponding set of bit lines.

Typically, the row selector of the flash memory includes low voltage predecoding circuits and high voltage decoding circuits.

The predecoding circuits operate with logical signals at low voltages, of the order of a supply voltage of the flash memory device (such as, 3V).

For this reason, the predecoding circuits can be implemented with low voltage components that are designed in such a way to be able to sustain (between their terminals) voltage differences that are up-limited (in absolute value) by the supply voltage. Indeed, the low voltages that are experienced by those components allow their correct functioning, without causing breaking thereof. For example, those components are low voltage MOSFETs, which are designed in such a way to avoid the occurrence of gate oxide breaking or undesired junctions breakdown when low voltage differences of the order of the supply voltage are applied to their terminals (for example, between the gate and source terminals).

The high voltage decoding circuits (for example including level shifters for shifting the signals which are necessary for the selection of the word lines during the operation to be performed on the memory flash) have to be able to apply operative voltages of high value to the selected memory cells (during program, read and erase operations). These voltages (for example, ranging from -9V to 9V) are higher (in absolute value) than the supply voltage. For example, in single supply voltage memory devices, the high voltages are generated inside the flash memory device from the supply voltage, by means of suitable circuits (such as, charge pumps).

For this purpose, the high voltage decoding circuits are implemented so as to manage the high voltages necessary during the program, read and erase operations; for example, during a program operation the row selector has to apply a programming voltage (such as, 9V) to the selected word line.

Therefore, the decoding circuit includes components that are designed in such a way to be able to sustain (between their terminals) voltage differences that are higher than the supply voltage (up to 9V in the cited example). For example, those components are high voltage MOSFETs, which are MOSFETs designed in such a way to avoid the occurrence of gate oxide breaking or undesired junctions breakdown even when high voltages (higher than the supply voltage) are applied to their terminals.

The high voltage transistors have a gate oxide layer thicker than that used for the low voltage transistors. Indeed, the thicker the gate oxide layer the higher the voltage sustained at their terminals without undesired breaking is. Since the high voltage transistors occupy more silicon area compared to the low voltage transistors, the row selector wastes a significant area of a chip wherein the flash memory device is integrated.

Moreover, the use of both low and high voltage transistors increases the number of processing steps and masks (for example, for differentiating the oxide thickness of the high and low voltage transistors); this has a detrimental impact on the manufacturing process of the flash memory device.

### Summary of the invention

In its general terms, the present invention is based on the idea of using components working at reduced voltage.

Particularly, the present invention provides a solution as set out in the independent claims.

Advantageous embodiments of the invention are provided in the dependent claims.

In detail, an aspect of the present invention proposes a row selector for a semiconductor memory. The semiconductor memory includes a plurality of memory cells coupled to a corresponding plurality of word lines. The row selector comprises for each word line: a first biasing circuit path adapted to bias the corresponding word line to a programming voltage when said corresponding word line is selected for selectively performing a program operation on at least one memory cell coupled to the corresponding word line, the first biasing circuit path comprising programming voltage provisioning means adapted to provide the programming voltage; a second biasing circuit path which is adapted to receive, from program-inhibit voltage provisioning means a program inhibit voltage, and to provide to the corresponding word line said program inhibit voltage when the word line is unselected during the program operation, first biasing means for driving the second biasing circuit path in order to control a conduction state thereof; wherein said first biasing circuit path includes a first transistor controlled to be electrically conductive when the corresponding word line is selected, and to be electrically non-conductive when the corresponding word line is unselected; said first biasing means controls the second biasing circuit path to be conductive when, during the program operation, the corresponding word line is unselected. The second biasing circuit path includes a plurality of series-connected transistors, a number of transistors in said plurality being at least equal to the smallest integer not less than an absolute value of a ratio between a voltage equal to the difference between the programming voltage and the program-inhibit voltage to a predetermined maximum voltage.

The features, and advantages of the present invention will be made apparent by the following detailed description of an embodiment thereof, provided merely by way of non-limitative example, description that will be conducted making reference to the attached drawings.

### Brief description of the drawings

**Figure 1** is a schematic block diagram of a memory device in which the solution according to an embodiment of the invention can be used, limitedly to functional blocks thereof relevant to the understanding of the present invention;
**Figure 2** schematically shows a row selector according to an embodiment of the present invention;
**Figure 3A** schematically shows the row selector of **Figure 2** during a program operation according to an embodiment of the present invention;
**Figure 3B** schematically shows the row selector of **Figure 2** during a read operation according to an embodiment of the present invention;
**Figure 3C** schematically shows the row selector of **Figure 2** during an erasing operation according to an embodiment of the present invention;
**Figure 4** is an exemplary implementation of a single word line selector block of the row selector of **Figure 2** according to an embodiment of the invention;
**Figure 5** shows a table listing voltage values of some internal nodes of the single word line selector block of **Figure 4** during the operation thereof according to an embodiment of the invention;
**Figure 6** schematically shows an exemplary implementation of a first biasing block of the row selector of **Figure 2** according to an embodiment of the invention;
**Figure 7** shows a table listing voltage values of the some internal nodes of the first biasing block of **Figure 6** during the operation thereof according to an embodiment of the invention;
**Figure 8** is an exemplary implementation of a second biasing block of the row selector of **Figure 2** according to an embodiment of the invention; and
**Figure 9** shows a table listing voltage values of some internal nodes of the second biasing block of the **Figure 8** during the operation thereof according to an embodiment of the invention.

### Detailed description of the preferred embodiment(s)

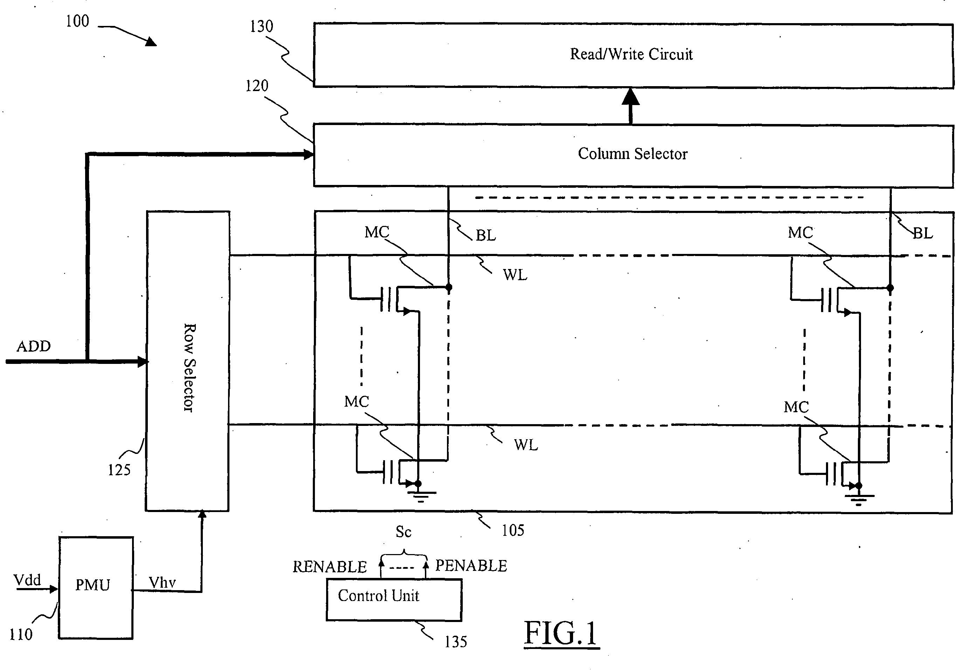

Referring to **Figure 1****,** a flash memory device **100** is schematically represented. The memory device **100** includes one or more sectors **105** (only one shown in the Figure). The sector **105** includes a plurality of memory cells **MC,** each one consisting of a floating gate MOSFET. In particular, the memory device **100** is of the flash type, and an erase operation affects all the memory cells **MC** of the generic sector **105.**

In an erased condition each memory cell **MC** has a low threshold voltage (to which a logic level "1" is typically associated). The memory cell **MC** is programmed by injecting electrons into its floating-gate; in this condition the memory cell **MC** has a high threshold voltage (to which a logic level "0" is typically associated).

In each sector **105,** the memory cells **MC** are arranged in rows and columns. The memory cells **MC** of each column have the drain terminals connected to a respective bit line **BL,** while the memory cells **MC** of each row have the control terminals connected to a respective word line **WL.** The source terminal of each memory cell **MC** receives a reference voltage **GND** (or ground) (alternatively, the source terminals of all the memory cells **MC** of a same sector are connected to a common source line, whose voltage may be properly varied depending on the operation to be performed).

The memory device **100** further includes a PMU (acronym for Power Management Unit) **110.** The PMU **110** provides the biasing voltages that are used for performing the conventional operations (such as read, program, erase and verify) on the memory device **100.** The PMU **110** receives a supply voltage **Vdd** (such as 3V) from the outside of the memory device, and outputs different operative voltages **Vhv;** the operative voltages **Vhv** are generally higher in absolute value than the supply voltage **Vdd,** for example, ranging from -9V to 9V. For this purpose, the PMU **110** includes a circuitry (*e.g.*, charge pumps) adapted to generate the operative voltages **Vhv** from the supply voltage **Vdd.** Preferably, those charge pumps are implemented by means of low voltage transistors only, as described in Patent Application No. EP05111284.5 filed on 25 November 2005in the name of the present Applicant.

The memory device **100** receives an addressing code **ADD** for accessing the memory cells **MC.** The addressing code **ADD** consists of a set of bits (such as 32). A portion of the addressing code **ADD** is supplied to a column selector **120,** which selects a set of desired bit lines **BL** (such as 8 or 16 bit lines at a time). Another portion of the addressing code **ADD** is supplied to a row selector **125,** which selects one desired word line **WL** at a time.

The column selector **120** couples the selected bit lines **BL** to a read-write circuit **130.** The read/write circuit **130** includes all the components (*e.g.*, sense amplifiers, comparators, reference current/voltage generators, pulse generators, program load circuits and the like), which are normally required for writing the desired logic values into the selected memory cells **MC** and for reading the logic values currently stored therein. The read/write circuit **130** is coupled to externally-accessible terminals of the memory device **100** (not shown in figure) for receiving/delivering data.

The row selector **125** receives the corresponding portion of the addressing code **ADD** and the operative voltages **Vhv.** According to the operation to be performed on the memory device **100,** the row selector **125** biases the word lines of the sector **105** to one or more operative voltages **Vhv** or the supply voltage **Vdd** or ground. For example, during a programming operation the row selector **125** biases a selected word line to one of the operative voltages **Vhv,** such as a programming voltage **POSV** (for example, **POSV** = 9V), whereas the remaining word lines are brought to the reference voltage **GND.** During an erasing operation, the row selector **125** biases all the word lines **WL** of the sector **105** to a different operative voltage **Vhv,** such as an erasing voltage **NEGV** (for example, **NEGV=** -9V). During a read operation, the row selector **125** biases a selected word line to one of the operative voltages **Vhv,** such as a reading voltage (for example, of 6V), whereas the remaining word lines are brought to the reference voltage **GND.**

The memory device **100** includes a control unit **135** which is adapted to provide control signals (denoted as whole as **Sc**) which are used for driving the components of the memory device during the operation thereof . For example, in order to perform the various operations on the memory cells **MC** of the memory device **100,** the control signals **Sc** include a programming enable signal **PENABLE** which controls the program operation and a reading enable signal **RENABLE** which controls the reading operation.

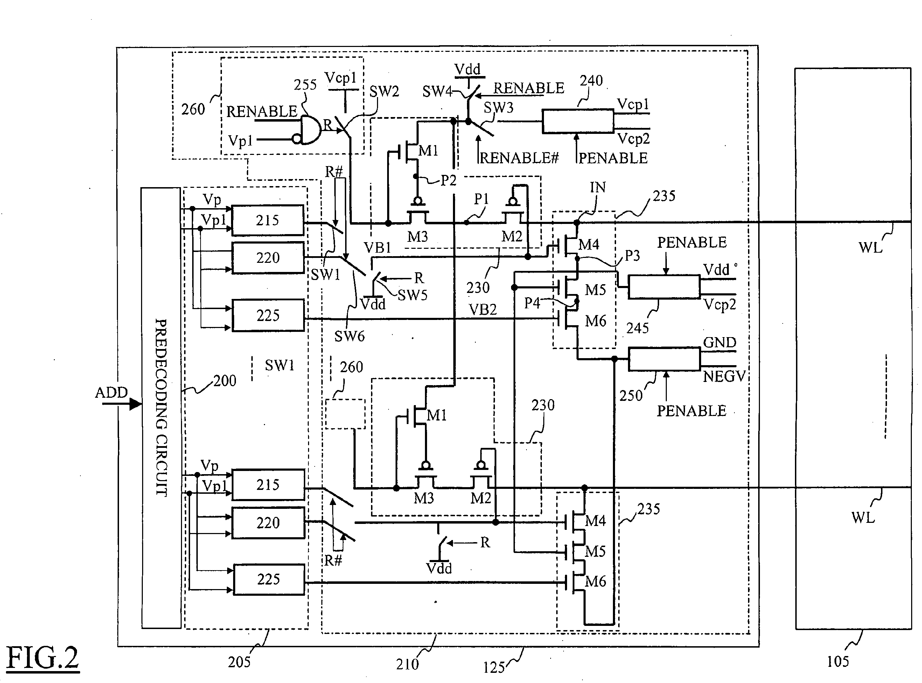

Referring to **Figure 2****,** a schematic implementation of the row selector **125** is shown. The row selector **125** includes a predecoding circuit **200,** which receives the corresponding portion of the addressing code **ADD** and generates a first (logical) selection signal **Vp** and a second (logical) selection signal **Vp1** for each word line **WL.**

Moreover, the row selector **125** includes a decoding circuit **205** which is coupled to the word lines of the sector **105** by means of an intermediate circuitry **210.** The intermediate circuitry **210** receives from the decoding circuit **205** one or more operative voltages **Vhv** and in response is able to bias one or more word lines **WL** of the sector **105** according to the operation to be performed on the memory device.

More in detail, the decoding circuit **205** includes for each word line **WL** of the sector **105** a single word line selector block **215,** which selects a corresponding word line during the operation of the memory device. In particular, each single word line selector block **215** receives the corresponding first selection signal **Vp** and the second selection signal **Vp1** and according to the assertion state thereof biases, by means of the intermediate circuitry **210,** the corresponding word line **WL** to one of the operative voltages **Vhv.**

Moreover, for each word line **WL** of the sector **105,** the decoding circuit **205** includes, a first biasing block **220** and a second biasing block **225** that are adapted for biasing one or more corresponding electronic components included in the intermediate circuitry **210.** The first biasing block **220** and the second biasing block **225** receive as well the corresponding first selection signal **Vp** and the second selection signal **Vp1.**

The intermediate circuitry **210** includes for each word line **WL** a first circuital structure **230** and a second circuital structure **235.** The first circuital structure **230** is adapted for coupling the word line **WL** to the corresponding single word line selector block **215** whereas the second circuital structure **235** is a circuit branch controlled by the first biasing block **220** and the second biasing block **225.**

The first circuital structure **230** includes a n-channel MOSFET **M1** and two p-channel MOSFETs **M2** and **M3.** The transistors **M2** and **M3** are connected in series, in particular the transistor **M3** has a first terminal adapted to be coupled to an output terminal of the corresponding single word line selector block **215** through a first switch **SW1.** Depending on the operation to be conducted, the first terminal of the transistor **M3**, in alternative to being coupled to the single word line selector block **215,** is adapted to receive one of the operative voltage **Vhv** (for example, a first biasing voltage **Vcp1**) through a first reading circuit **260.** For example, the first biasing voltage **Vcp1** is a voltage of approximately 6V, used for biasing a word line containing a memory cell to be read.

In detail, the first reading circuit **260** includes a second switch **SW2** controlled by an enabling signal **R,** generated by an AND gate **255** which receives a complemented second selection signal **Vp1#** and the reading enable signal **RENABLE.** The first switch **SW1** is enabled by a complemented enabling signal **R#.** In other words, when the complemented enabling signal **R#** is asserted (to the supply voltage **Vdd**) the first switch **SW1** is closed.

A second terminal (denoted as node **P1**) of the transistor **M3** is connected to the first terminal of the transistor **M2.** The transistor **M2** has the second terminal (denoted as intermediate node **IN**) which is coupled to the corresponding word line **WL.** The control terminal of the transistor **M3** is connected to the second terminal of the transistor **M1** (denoted as node **P2**), whereas the first terminal of the transistor **M3** is connected to the control terminal of the transistor **M1.** The first terminal of the transistor **M1** receives one of operative voltages **Vhv** by means of a first voltage switch **240.** In particular, the first terminal of the transistor **M1** is coupled to the first voltage switch **240** through a third switch **SW3,** controlled by the complemented reading enable signal **RENABLE**#**.** Moreover, the first terminal of the transistor **M1** is adapted to receive the supply voltage **Vdd** trough a fourth switch **SW4** controlled by the reading enable signal **RENABLE.**

The first voltage switch **240** is a circuit adapted to selectively connect one input terminal, selected among two its input terminals each one adapted to receive a respective operative voltage **Vhv** (for example, the first biasing voltage **Vcp1** = 6V and a second biasing voltage **Vcp2** = -6V), to a switch output terminal which is connected (when the third switch **SW3** is closed) to the first terminal of the transistor **M1.** The first voltage switch **240** receives the program enable signal **PENABLE** and according to assertion state thereof selects the desired input terminal which has to be coupled to the first terminal of the transistor **M1**. More in particular, when the program enable signal **PENABLE** is asserted (at the supply voltage **Vdd**), the first voltage switch **240** provides at the output the first biasing voltage **Vcp1;** vice versa the first voltage switch **240** provides at the output the second biasing voltage **Vcp2**. Preferably, the voltage switch **240** is implemented by means of low voltage transistors only, as described in the co-pending Patent Application No.EP06111477.3 filed on 21/March /2006.

The second circuital structure **235** is a circuit branch including three n-channel MOSFETs **M4, M5** and **M6** in series. More in particular, the transistor **M4** has the second terminal connected to the second terminal of the transistor **M2** (*i.e.,* the intermediate node **IN)** and the first terminal connected to the second terminal of the transistor **M5** (denotes as node **P3**). The transistor **M5** has the first terminal connected to the second terminal of the transistor **M6** (denoted as node **P4**) and the control terminal connected to a second voltage switch **245.** The second voltage switch **245** is a circuit adapted to selectively connect one between two input terminals thereof to a switch output terminal which is connected to the control terminal of the transistor **M5.** In particular, one input terminal is adapted to receive a corresponding operative voltage **Vhv** (for example, the second biasing voltage **Vcp2** = -6V) whereas the other one is adapted to receive the supply voltage **Vdd.** The second voltage switch **245** receives the program enable signal **PENABLE** and, according to assertion state thereof, selects the desired input terminal whose voltage is to be made available at the output. More in particular, when the program enable signal **PENABLE** is asserted (at the supply voltage **Vdd**), the second voltage switch **245** provides the supply voltage **Vdd** at the output; vice versa the second voltage switch **245** provides the second biasing voltage **Vcp2**.

The transistor **M6** has the first terminal connected to a switch output terminal of a third voltage switch **250.** The third voltage switch **250** has one input terminal which receives a corresponding operative voltage **Vhv** (for example, the erasing voltage **NEGV**= -9V) whereas the other one receives the reference voltage **GND.** Similarly to the voltage switches **240** and **245,** the voltage switch **250** receives the program enable signal **PENABLE** that commands the coupling of the desired input terminal to the first terminal of the transistor **M6.** In particular, when the program enable signal **PENABLE** is asserted (at the supply voltage **Vdd)** the third voltage switch **250** provides the reference voltage **GND;** on the contrary, the third voltage switch **250** provides the erasing voltage **NEGV.**

Moreover, the control terminal of the transistor **M4** is connected to a fifth switch **SW5** controlled by the enabling signal **R** and a sixth switch **SW6** controlled by the complemented enabling signal **R#.** In particular, when the enabling signal **R** is asserted (at the supply voltage **Vdd)** the switch **SW5** is closed and the sixth switch **SW6** is open.

The control terminal of the transistor **M6** receives from the second biasing block **220** a second control signal **VB2.**

As can be noted, the intermediate circuitry **210** includes the above described first and second circuit structures for each word line.

During the program operation, the memory device receives the addressing code for accessing the memory cell(s) to be programmed. In particular, for selecting the word line, the corresponding first selection signal **Vp** is asserted (at the supply voltage **Vdd)** whereas the corresponding second selection signal **Vp1** is de-asserted (at the reference voltage **GND).** For the remaining unselected word lines the corresponding first and second selection signals **Vp** and **Vp1** are both asserted. At the same time, the control unit **135** asserts the programming enable signal **PENABLE** and deasserts the reading enable signal **RENABLE.** The switch **SW4** is open, whereas the switch **SW3** is closed; the enabling signal **R** is deasserted (at the reference voltage **GND**), so the switches **SW1, SW6** are closed and the switches **SW2** and **SW5** are open.

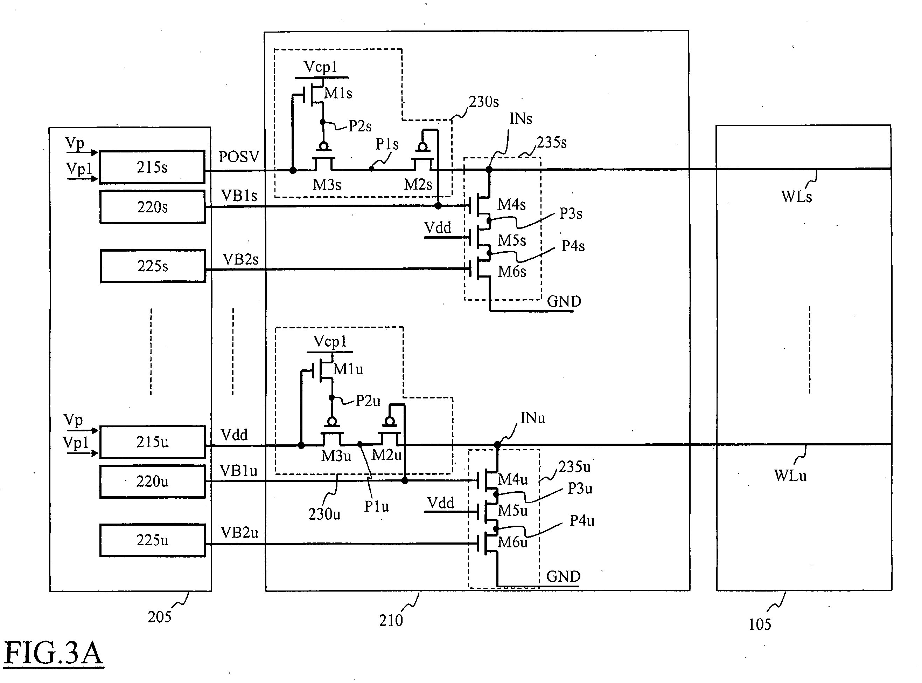

As above discussed, the row selector **125** is able to select the desired word line **WL** and to bias it at the proper voltage in order to perform the program operation. For this purpose, as shown in **Figure 3A****,** two word lines are considered: a selected word line **WLs** and an unselected word line **WLu** (from now on, the suffixes "s" and "u" will be appended to all references of the components included in the row selector **125** to discriminate those associated or related to selected word lines from those associated or related to unselected word lines). Moreover, in all the exemplificative cases illustrated in the following, both the p-channel MOSFETs and the n-channel MOSFETs included in the row selector **125** are assumed to have a threshold voltage of 1 Volt (in absolute value), and the supply voltage **Vdd** is assumed to be equal to 3 Volts with respect to the reference voltage **GND.**

The single word line selector block **215s** associated with the selected word line and responsive to the selection signals **Vp** and **Vp1** provides one of the operative voltages **Vhv** such as the programming voltage **POSV** (for example, **POSV** = 9V) to the intermediate circuit **210.** In particular, the programming voltage **POSV** is provided to the first terminal of the transistor **M3s** and to the control terminal the transistor **M1s.** Moreover, since the program control signal **PENABLE** is asserted, the first terminal of the transistor **M1s** receives the first biasing voltage **Vcp1** (for example, **Vcp1** = 6V) by means of the first voltage switch **240.**

The second circuital structure **235s** receives by means of the first and the second biasing blocks **220s** and **225s** the first and second control signals **VB1** and **VB2** at the voltages **VB1s** and **VB2s,** respectively. For example, **VB1s** = 6V and **VB2s** = 0V.

Moreover, since the program control signal **PENABLE** is asserted, by means of the corresponding second and third voltage switches **245** and **250,** the transistor **M5s** receives at its control terminal the supply voltage **Vdd** and the transistor **M6s** receives to its first terminal the reference voltage **GND.**

During the operation, the intermediate node INs is brought to the programming voltage **POSV.** More in particular, the transistor **M1s** is turned on since its driving voltage (between the control and first terminal) is equal to the supply voltage **Vdd.** On the other hand, the transistor **M1s** does not conduct any current since it is connected in series to the control terminal of the transistor **M3s** which has a significantly high resistance. Thus, the voltage of the node **P2s** (and consequently the voltage of the control terminal of the transistor **M3s)** reaches the biasing operative voltage **Vcp1** (in the example at issue, 6V). In such a way, the transistor **M3s** is turned on (because its driving voltage is equal to the supply voltage **Vdd),** and the node **P1s** is brought to the programming voltage **POSV.** In this biasing condition, the transistor **M2s** is turned on as well (because its driving voltage, equal to the difference between the voltage **VB1s** and the voltage **POSV** is equal to the supply voltage **Vdd),** thus biasing the selected word line **WLs** to the programming voltage **POSV**. In other words, the first circuital structure **230s** forms a conductive path which is adapted for biasing the selected word line **WLs** at the programming voltage **POSV**.

In the second circuital structure **235s,** the transistor **M6s** is turned off since its driving voltage is equal to zero (the voltage **VB2s** is equal to ground). The transistors **M4s** and **M5s** are connected in series to the transistor **M6s,** thus both the transistors **M4s** and **M5s** cannot conduct any current because the transistor **M6s** is turned off. In this biasing condition, the transistors **M4s, M5s** and **M6s** do not interfere with the voltage of the intermediate node **INs.** In other words, the transistors **M4s, M5s** and **M6s** form a path with a significant high resistance, thus allowing to insulate the intermediate node **INs** from the reference voltage **GND.**

More in detail, the voltage of the node **P3s** is at most equal to the voltage of first control signal **VB1** (*i.e.,* in the example, the voltage **VB1s** = 6V) minus the threshold voltage of the transistor **M4s** (in the example at issue, 6-1 = 5V), otherwise the transistor **M4s** should be conductive. Likewise, the voltage of the node **P4s** is at most equal to the supply voltage **Vdd** minus the threshold voltage thereof (in the example at issue, 3-1 = 2V), otherwise the transistor **M5s** should be conductive.

As can be noted, the transistors **M1s, M2s** and **M3s** of the first circuital structure **230s** and the transistors **M4s, M5s** and **M6s** of the second circuital structure **235s** sustain between their control and first/second terminals a voltage difference at most equal to the supply voltage **Vdd.** In other words, the transistors **M1s - M6s** are low voltage type transistors (*i.e*., the circuital structures **230s** and **235s** can be implemented with low voltage transistors only, without having to use high-voltage transistors).

Referring now to unselected word line **WLu,** the remaining word line selector block **215u** provides the supply voltage **Vdd** to the intermediate circuit **210** (and thus to the corresponding first terminal of the transistor **M3u** of the first circuital structure **230u**). In this case the first and the second selection signals **Vp** and **Vp1** are asserted (at the supply voltage **Vdd**).

The second circuital structure **235u** receives the control signals **VB1** and **VB2** at the voltages **VB1u** and **VB2u**, respectively. For example, **VB1u** = **VB2u** = 3V.

Moreover, since the program control signal **PENABLE** is asserted, the first terminal of the transistor **M1u** receives the first biasing voltage **Vcp1** by means of the first voltage switch **240.** Likewise, by means of the corresponding voltage switches **245** and **250,** the transistor **M5u** receives at its control terminal the supply voltage **Vdd** and the transistor **M6u** receives to its first terminal the reference voltage **GND.**

During the program operation, the intermediate node **INu** (and thus the unselected word line **WLu**) is brought to the reference voltage **GND.** For this purpose, the second biasing block **225u** provides the second control signal **VB2** at the value (in the example, **VB2u** = 3V) adapted to turn on the transistor **M6u** of the second circuital structure **235u** (in such a way, the transistor **M6u** has its driving voltage equal to the supply voltage **Vdd**). In this case, the node **P4u** reaches approximately the reference voltage **GND,** thereby allowing to the transistor **M5u** to turn on (since its driving voltage is equal to the supply voltage **Vdd**). Thus, the node **P3u** reaches the reference voltage **GND.** The transistor **M4u** is turned on (since its driving voltage is equal to the supply voltage **Vdd,** being the voltage **VB1u** equal to 3V, in this example) so as to bring the intermediate node **INu** to the reference voltage **GND.** In other words, the second circuital structure **235u** forms a conductive path which is adapted to bring the unselected word line **WLu** to ground.

Also in this case, the transistors **M4u, M5u** and **M6u** sustain between their control and first/second terminals a voltage difference at most equal to the supply voltage **Vdd.** In other words, the transistors **M4u, M5u** and **M6u** can be low voltage type transistors.

Moving to the first circuital structure **230u,** the transistor **M2u** is turned off (since its driving voltage is higher than its threshold voltage), thereby isolating the intermediate node **INu** from the voltage provided by the single word line selector block **215u.** The transistor **M2u** is connected in series to the transistor **M3u.** In such a way, the transistor **M3u** cannot conduct any current and the first circuital structure **230u** is adapted to form a path with a significant high resistance. The transistor **M1u** is turned off, because its driving voltage is lower than threshold voltage thereof.

Also in this case, the voltage differences sustained across the terminals of all transistors included in the first circuital structure **230u** are at most equal to the supply voltage **Vdd.** Therefore, the transistors **M1u**, **M2u** and **M3u** can be low voltage type transistors.

Therefore, thanks to the row selector structure described above, it is possible to avoid the use of high voltage transistors having relatively thick oxide layers (capable to sustain across their terminals voltages higher than the supply voltage **Vdd**). This reduces significantly the area occupied by the memory device and the number of processing steps and masks of the manufacturing process of the memory device.

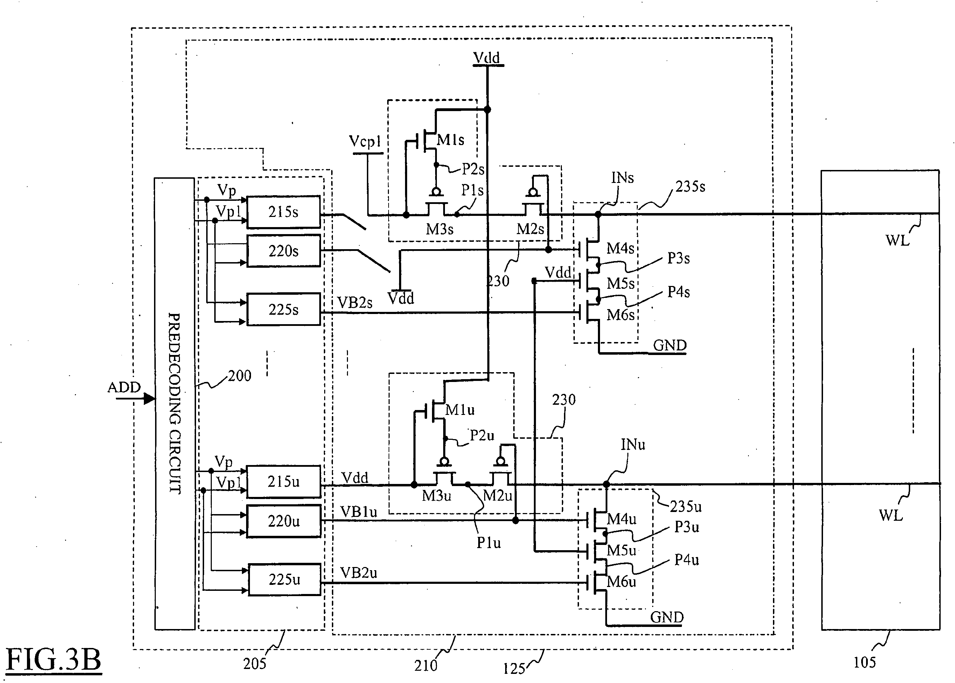

Moving to **Figure 3B****,** during a reading operation, the control unit **135** asserts the reading enable signal **RENABLE** and the programming enable signal **PENABLE.** In this biasing condition, the first terminals of the transistors **M1s** and **M1u** and the control terminal of the transistor **M4s** (related to selected word line **WLs)** receive the supply voltage **Vdd.** Moreover, the first terminal of the transistor **M3s** receives the first operative voltage **Vcp1** (in the example at issue equal to 6V).

In this biasing condition, the transistor **M1s** is turned on (since its driving voltage is equal to the supply voltage **Vdd)** thereby the node **P2s** is brought to the supply voltage **Vdd.** In such a way, the transistor **M3s** is turned on (since its driving voltage is equal to the supply voltage **Vdd**), so that the node **P1s** is brought to the first operative voltage **Vcp1.** The transistor **M2s** is turned on, so that the intermediate node **INs** reaches the first operative voltage **Vcp1.** Similarly to the program operation, the second circuital structure **235s** forms a non-conductive path, because the transistor **M6s** is turned off, with the nodes **P3s** and **P4s** which can reach at most the supply voltage **Vdd** minus the threshold voltage of the transistor **M4s** and **M5s,** respectively.

Like in the program operation, the second circuital structure **235u** forms a conductive path, so the word line **WLu** is brought to the reference voltage **GND.** Concerning the first circuital structure **230u,** the transistor **M2u** is turned off (since its driving voltage is lower than its threshold voltage), thereby isolating the intermediate node **INu** from the voltage provided by the single word line selector block **215u.** The transistor **M2u** is connected in series to the transistor **M3u,** which cannot conduct any current, and the first circuital structure **230u** is adapted to form a high-resistance path. The transistor **M1u** is turned off (since its driving voltage is lower than threshold voltage thereof). Also in this case, all the transistors sustain voltage differences (between the control terminal and other terminals thereof) at most equal to the supply voltage **Vdd.**

During the erasing operation, the whole selected memory sector **105** is erased. For selecting all the word lines of the sector to be erased, each of the first selection signals **Vp** and each of the second selection signals **Vp1** corresponding to the word lines of the sector are deasserted (at the reference voltage **GND**). At the same time, the control unit **135** deasserts the program enable signal **PENABLE** and the reading enable signal **RENABLE.**

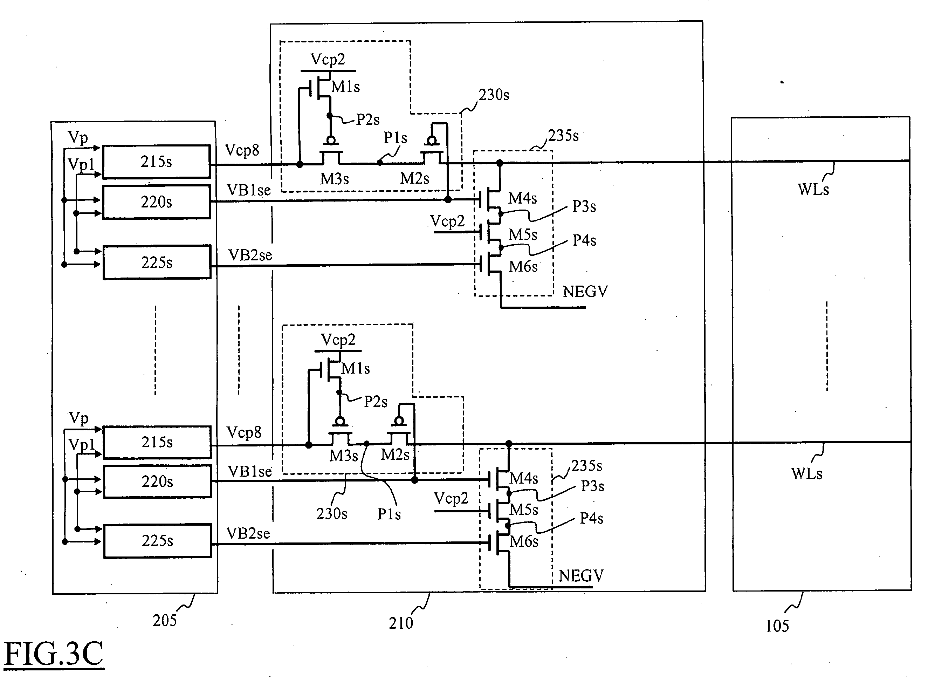

In other words, as shown in **Figure 3C****,** the row selector **125** selects all the word lines (for this reason all the word lines in the sector are denoted with the reference **WLs**), and biases them to the erasing voltage **NEGV** (in the example at issue, **NEGV** = -9V).

Since the program control signal **PENABLE** is deasserted, each transistor **M1s** receives to its first terminal the second biasing voltage **Vcp2** (for example, **Vcp2** = -6V) through the first voltage switch **240.** Likewise, the second and third voltage switches **245** and **250** provide to the control terminal of each transistor **M5s** and to the first terminal of each transistor **M6s** the second biasing voltage **Vcp2** (*e.g.*, **Vcp2** = -6V) and the erasing voltage **NEGV** (*e.g.,* **NEGV** = -9V), respectively. The first and second control signals **VB1** and **VB2** reach values **VB1se** and **VB2se** (for example, **VB1se** = **VB2se**= -6V). Moreover, each single word line selector block **215s** provides to the first terminal of the associated transistor **M3s** one of the operative voltages, for example a voltage **Vcp8** (for example, **Vcp8** = -6V).

In this biasing condition, each second circuital structure **235s** forms a conductive path that brings the corresponding word line to the erasing voltage **NEGV.** In particular, the transistor **M6s** is turned on (since its driving voltage is higher than its threshold voltage). In such a way, the node P4s reaches approximately the erasing voltage **NEGV,** thus turning on the transistor **M5s.** In such a way, the node **P3s** reaches the erasing voltage **NEGV,** so that also the transistor **M4s** is turned on: the intermediate node **INs** is thus brought to the erasing voltage **NEGV.**

As can be noted, the transistors **M4s, M5s** and **M6s** sustain a voltage differences (between the control terminal and any other terminals thereof) at most equal to the supply voltage **Vdd,** so they can be low voltage transistors.

Moving to the first circuital structure **230s,** the transistor **M2s** is turned off (since its driving voltage is lower than its threshold voltage). The transistor **M2s** is connected in series to the transistor **M3s,** which cannot conduct any current, and the first circuital structure **230s** is adapted to form a high-resistance path , thereby insulating the intermediate node **INs** from the voltage provided through the corresponding single word line block selector **215s.** Also in this case, the voltage differences sustained at the terminals of all transistors included in the first circuital structure **230s** are at most equal to the supply voltage **Vdd** and the transistors **M1s**, **M2s** and **M3s** can be low voltage transistors (with the advantages discussed in the foregoing).

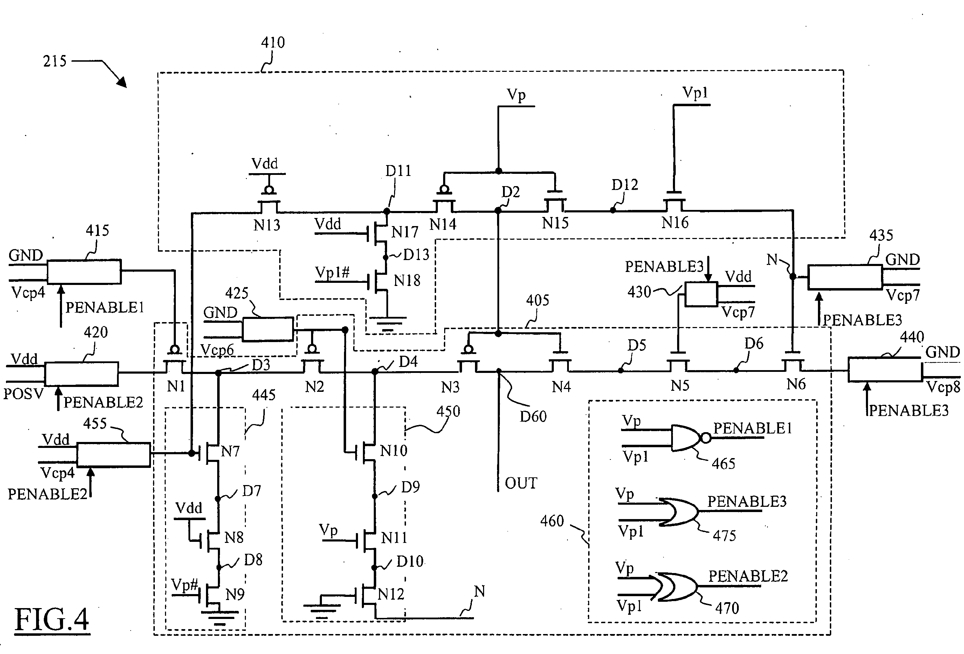

Referring to **Figure 4****,** an exemplary implementation of a generic single word line selector block **215** is schematically shown. The single word line selector block **215** includes a first and a second circuital blocks **405** and **410.**

The first circuital block **405** includes a first circuit branch with three p-channel MOSFETs **N1, N2** and **N3** connected in series, and a second circuit branch with three further series-connected n-channel MOSFETs **N4, N5** and **N6,** the first and second circuit branches being both connected to a common node **D60.** In particular, the transistor **N1** has the control terminal connected to an output terminal of a fourth voltage switch **415** and a first terminal connected to an output terminal of a fifth voltage switch **420.** The fourth voltage switch **415,** having two input terminals each one adapted to receive one of the operative voltages **Vhv** (for example, a fourth biasing voltage **Vcp4** = 6V) and the reference voltage **GND** respectively, selectively connects one of its input terminals to the control terminal of the transistor **N1,** according to the operation to be performed on the memory device. The voltage switch **415** receives a first control signal **PENABLE1** that, according to its assertion state, causes the selection of a corresponding switch input terminal. In particular, when the control signal **PENABLE1** is asserted (at the supply voltage **Vdd**), the voltage switch **415** provides at the switch output the operative voltage Vcp4; on the contrary, the voltage switch **415** provides the reference voltage **GND.**

Likewise, the fifth voltage switch **420,** having two input terminals each one adapted to receive one of the operative voltages **Vhv** (for example, the programming voltage **POSV**) and the supply voltage **Vdd**, selectively connects one of its input terminals to the first terminal of the transistor **N1** according to the operation to be performed on the memory device. The voltage switch **420** receives a second control signal **PENABLE2,** whose assertion state causes the selection of either one of the switch input terminals. In particular, when the control signal **PENABLE2** is asserted (at the supply voltage Vdd), the voltage switch **420** provides at the switch output the programming voltage **POSV;** on the contrary, the voltage switch **420** provides the supply voltage **Vdd**.

The second terminal of the transistor **N1** (denoted as node **D3**) is connected to a first terminal of the transistor **N2,** which has the second terminal (denoted as node **D4**) connected to a second terminal of the transistor **N3.** A sixth voltage switch **425** is adapted for driving the transistor **N2.** In particular, the control terminal of the transistor **N2** is connected to an output terminal of the voltage switch **425.** The voltage switch **425** has two input terminals each one adapted to receive an operative voltage **Vhv** (for example, the fourth biasing voltage Vcp4 = 6V) and the reference voltage **GND,** respectively; moreover, the voltage switch **425** receives the second control signal **PENABLE2.** Each one of the input terminals of the voltage switch **425** is adapted to be connected to the switch output terminal, and, according to the assertion state of the control signal **PENABLE2,** the transistor **N2** can alternatively be driven by the operative voltage Vcp4 or the reference voltage **GND.** The first terminal of the transistor **N3** is connected to the node **D60;** the node **D60** is connected to an output terminal of the word line selector block **215** which is adapted for providing a word line selecting signal **OUT.** The node **D60** is also connected to a second terminal of the transistor **N4,** which has the control terminal connected to the control terminal of the transistor **N3.** Moreover, the transistor **N4** has a first terminal (denoted as node **D5**) connected to a second terminal of the transistor **N5.** The transistor **N5** has a control terminal which is connected to an output terminal of a seventh voltage switch **430.** The voltage switch **430** has two input terminals which receive a respective operative voltage **Vhv,** for example, the seventh biasing voltage **Vcp7** = -3V and the supply voltage **Vdd,** respectively. Moreover, the seventh voltage switch **430** receives a third control signal **PENABLE3** that, according to its assertion state, causes the connection of one of the switch input terminals to the switch output terminal, thereby the transistor **N5** can be alternatively driven by the operative voltage **Vcp7** or the supply voltage **Vdd.** In particular, when the third control signal **PENABLE3** is asserted, the voltage switch **430** provides the supply voltage **Vdd;** vice versa, the voltage switch **430** provides the operative voltages **Vcp7.**

A first terminal of the transistor **N5** (denoted as node **D6**) is connected to a second terminal of the transistor **N6.** A control terminal of the transistor **N6** is connected to an output terminal of an eighth voltage switch **435.** The voltage switch **435** has two input terminals each one adapted to receive one of the operative voltages **Vhv,** for example, the seventh biasing voltage **Vcp7** and the reference voltage **GND,** respectively. Similarly to the voltage switch **430,** the voltage switch **435** receives the third control signal **PENABLE3** that, according to its assertion state, causes the switch to connect one of its input terminals to the output terminal thereof, thereby the transistor **N6** can be alternatively driven by the operative voltage **Vcp7** or the reference voltage **GND.** In particular, when the third control signal **PENABLE3** is asserted, the voltage switch **435** provides the reference voltage **GND;** on the contrary, the voltage switch **435** provides the operative voltages **Vcp7.**

A first terminal of the transistor **N6** is connected to a ninth voltage switch **440.** The voltage switch **440** has two input terminals, one of which receives one of the operative voltages **Vhv,** for example, the voltage **Vcp8** = -6V, and the other one receives the reference voltage **GND.** Each one of the input terminals of the voltage switch **440** is alternatively connected to the switch output terminal according to the assertion state of the control signal **PENABLE3,** thus allowing to bring the first terminal the transistor **N6** at the operative voltage **Vcp8** or at the reference voltage **GND.** In particular, when the third control signal **PENABLE3** is asserted, the voltage switch **440** provides at the switch output the reference voltage **GND;** on the contrary, the voltage switch **440** provides the operative voltages **Vcp8.**

The first circuital block **405** further comprises two circuital structures **445** and **450.**

In detail, the circuital structure **445** includes three n-channel MOSFETs **N7, N8** and **N9** which are connected in cascade. In particular, the transistor **N7** has a second terminal connected to the second terminal of the transistor **N1,** and the first terminal (denoted as node **D7**) connected to a second terminal of the transistor **N8.** The transistor **N7** has a control terminal which is connected to an output terminal of a tenth voltage switch **455.** The voltage switch **455** has two input terminals, respectively adapted to receive the fourth biasing voltage **Vcp4** = 6V and the supply voltage **Vdd.** The voltage switch **455** is controlled by the second control signal **PENABLE2,** and according to the assertion state thereof, one of the switch input terminals is connected to the switch output terminal, so that the control terminal of the transistor **N7** can be alternatively biased at the operative voltage **Vcp4** or at the supply voltage **Vdd**. The transistor **N8** has a first terminal (denoted as node **D8**) connected to a second terminal of the transistor **N9** and a control terminal which is adapted to receive the supply voltage **Vdd.** The transistor **N9** has a first terminal connected to ground, whereas its control terminal receives a complemented first selection signal **Vp#.**

The circuital structure **450** includes three n-channel MOSFETs **N10, N11** and **N12** connected in cascade. In particular, the transistor **N10** has a second terminal connected to the node **D4,** and a first terminal (denoted as node **D9)** connected to a second terminal of the transistor **N11.** The transistor **N10** has a control terminal connected to the output terminal of the voltage switch **425.** The transistor **N11** has a first terminal (denoted as node **D10**) connected to a second terminal of the transistor **N12** and a control terminal adapted to receive the first selection signal **Vp.** The transistor **N12** has a first terminal connected to the output terminal (denoted as node **N**) of the fifth voltage switch **435** and a control terminal that receives the reference voltage **GND.**

The first circuital block **405** has an output terminal **D2** that is connected to the control terminal of the transistors **N3** and **N4.**

The second circuital block **410** includes a first circuit branch with two p-channel MOSFETs **N13** and **N14** connected in series, and a second circuit branch with two further series-connected n-channel MOSFETs **N15** and **N16,** the first and second circuit branches being both connected to the common node **D2.** In particular, the transistor **N13** has a first terminal connected to the output terminal of the voltage switch **455** and a control terminal which receives the supply voltage **Vdd.** The second terminal (denoted as node **D11**) of the transistor **N13** is connected to a first terminal of the transistor **N14.** The transistor **N14** has a second terminal and a control terminal connected to the first terminal and to the control terminal of the transistor **N15,** respectively. The control terminals of the transistors **N14** and **N15** are both driven by the first selection signal **Vp.** A second terminal of the transistor **N15** (denoted as node **D12**) is connected to a second terminal of the transistor **N16.** The transistor **N16** receives at its control terminal the second selection signal **Vp1,** whereas the first terminal of the transistor **N16** is connected to the output terminal of the voltage switch **435.**

Two n-channel MOSFETs **N17** and **N18** are connected in series between node **D11** and the ground. In particular, the transistor **N17** has a second terminal connected to node **D11** the control terminal which receives the supply voltage **Vdd,** and a first terminal (denoted as node **D13**) connected to a second terminal of the transistor **N18.** The transistor **N18** has a first terminal connected to ground and a control terminal adapted to receive the complemented signal **Vp1#.**

In the example at issue, the control signals **PENABLE1, PENABLE2** and **PENABLE3** are provided by a control circuit **460.** In particular, the first control signal **PENABLE1** is provided through an NAND gate **465** that receives the first selection signal **Vp** and the second selection signal **Vp1.** The second control signal **PENABLE2** is provided through an EX-OR gate **470** that receives the first selection signal **Vp** and the second selection signal **Vp1.** The third control signal **PENABLE3** is provided through a OR gate **475** that receives the first selection signal **Vp** and the second selection signal **Vp1.**

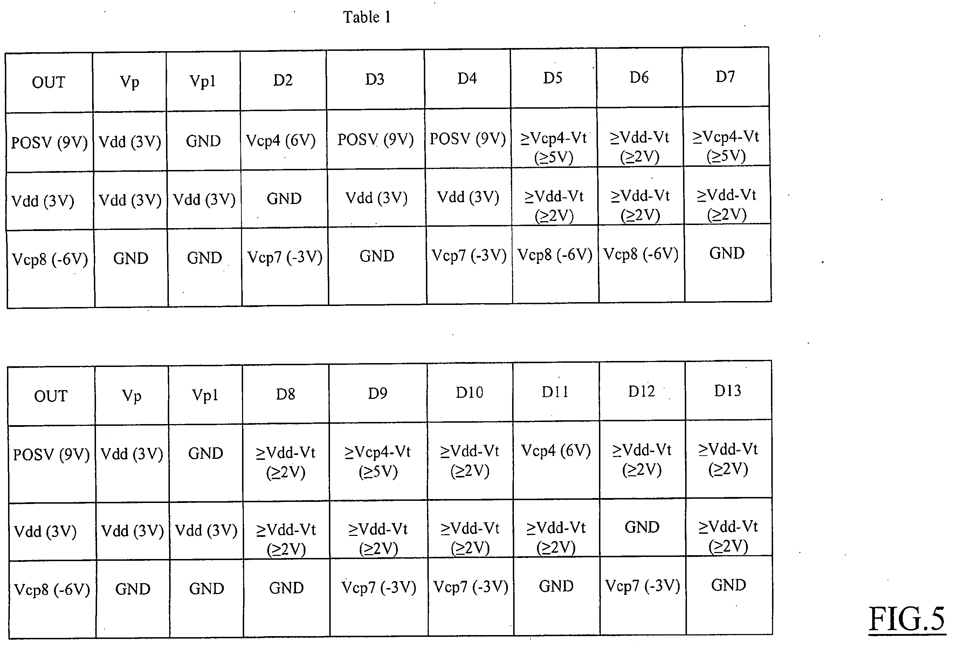

Referring now jointly to **Figures 4** and **5****,** a **Table 1** is shown in **Figure 5****,** reporting exemplary voltages of the most significant nodes of the single word line selector block **215** as a function of the operation to be performed on the memory device. In particular, according to the assertion state of the first selection signal **Vp** and the second selection signal **Vp1,** the word line selector block **215** brings the selecting signal **OUT** to the desired voltage during the operation to be performed; for example, during the programming operation the selecting signal **OUT** reaches the programming voltage **POSV** for the selected word line **WL** and the supply voltage **Vdd** for the unselected word lines. Whereas, during the erasing operation the selecting signal **OUT** reaches the operative voltage **Vcp8** (in the example at issue, Vcp8 = **-6V**). During the program operation, for selecting the word line to the memory cell(s) to be programmed belong(s) the first selection signal **Vp** is asserted (at the supply voltage **Vdd**) and the second selection signal **Vp1** is deasserted (at the reference voltage **GND**). In this condition, the control circuit **460** asserts the control signals **PENABLE1, PENABLE2** and **PENABLE3** (bringing them to the supply voltage **Vdd**).

The single word line selector block **215** associated with the selected word line operates as follows. Since the second control signal **PENABLE2** is asserted, the first terminal of the transistor **N13** receives the operative voltage **Vcp4** (for example, **Vcp4** = 6V). In this way, the transistor **N13** is turned on (since its driving voltage is higher than its threshold voltage), and the node **D11** thus reaches the fourth operative voltage **Vcp4** (in the example at issue, approximately equal to 6V). The transistor **N14** is turned on, as well (because its driving voltage is equal to the supply voltage **Vdd**), so the node **D2** is brought to 6V. The transistor **N18** is turned off (since its driving voltage is equal to zero), thus the transistor **N17** cannot conduct any current; in this biasing condition, the voltage of the node **D13** takes a value to not less than the supply voltage **Vdd** minus the threshold voltage of the transistor **N17** (in the example at issue, 3V - 1V = 2V) and does not interfere with the voltage of the node **D11.**

The transistor **N16** is turned off as well (since its driving voltage is equal to zero), so also the transistor **N15** cannot conduct any current. In this way, the node **D12** can reach the supply voltage **Vdd** minus the threshold voltage of the transistor **N15** (for example, the voltage of the node **D12** takes a value not less than 3V - 1V = 2V as shown in **Table 1**).

Moreover, since the first control signal **PENABLE1** is also asserted, the first terminal of the transistor **N1** receives the programming voltage **POSV** (for example, **POSV** = 9V), while its control terminal receives the fourth operative voltage **Vcp4.** In such a way, the transistor **N1** is turned on, and brings the node **D3** to the programming voltage **POSV.** Also the transistor **N2** is turned on (since it receives at its control terminal the fourth operative voltage **Vcp4**), so the node **D4** is brought to the programming voltage **POSV.** In such biasing condition, the transistor **N3** receives the supply voltage **Vdd** between the nodes **D4** and **D2,** and results turned on. In this way, the selecting signal **OUT** reaches the programming voltage POSV (which is equal to 9V, as shown in **Table 1**).

Since the control signal **PENABLE3** is asserted, the transistor **N6** has the first terminal and the control terminal both receiving the reference voltage **GND.** Moreover, the transistor **N5** has the control terminal which receives the supply voltage **Vdd.** In this biasing condition, the transistor **N6** is turned off (because its driving voltage is equal to zero), and then the transistors **N5** and **N4** cannot conduct any current. In particular, the nodes **D5** and **D6** reach the voltage of the control terminals of the transistors **N4** and **N5** minus the threshold voltage thereof, respectively (for example, the voltage of the node **D6** takes a value not less than 3V - 1V = 2V and the voltage of the node **D5** takes a value not less than 6V - 1V = 5V).

In the circuital structure **445,** the transistor **N9** is turned off (since its driving voltage is equal to zero). Thus, the transistors **N7** and **N8** cannot conduct any current, so the nodes **D8** and **D7** take a voltage value not less than to the voltage of their respective control terminals minus the threshold voltage (for example, the voltage of the node **D8** falls to approximately 3V - 1V = 2V and the voltage of the node **D7** falls to approximately 6V - 1V = 5V). Likewise, in the circuital structure **450** the transistor **N12** is turned off (since its driving voltage is equal to zero) and the transistors **N10** and **N11** cannot conduct any current, so the nodes **D9** and **D10** take a voltage value not approximately equal to the voltage of their control terminals minus the threshold voltage, respectively (for example, the voltage of the node **D10** takes a voltage value of approximately 3V-1V = 2V and the voltage of the node **D9** takes a voltage value approximately equal to 6V-1V = 5V). In such a way, the two circuital structures **445** and **450** do not interfere with the voltage of the nodes **D3** and **D4,** respectively.

As can be noted, during the program operation of the memory cell(s) corresponding to the selected word line, each transistor of the single word line block **215** sustains (between its control terminal and any other terminal thereof) voltage differences at most equal to the supply voltage **Vdd.**

As above described, during this operation the branch including the transistors **N4, N5** and **N6** and the branch including the transistors **N15** and **N16** are not conductive paths so do not interfere with the voltage reached by the node **D60** and **D2,** respectively. In this biasing condition, these branches are able to sustain a voltage drop equal to the programming voltage **POSV** and the operative voltage **Vcp4,** respectively, without breaking of low-voltage transistors thereof.

In a preferred embodiment of the invention both the programming voltage **POSV** and the operative voltage **Vcp4** are an integer multiple of the supply voltage **Vdd** (in the example at issue, **POSV** = **3Vdd** and **Vcp4** = **2Vdd**). In order to maintain not conductive the branch including the transistors **N4, N5** and **N6** and the branch including the transistors **N15** and **N16,** each branch includes a number of low voltage transistors connected in series equal to the integer multiple. In the example at issue, three low voltage transistors **N4, N5** and **N6** are provided in the branch adapted to sustain the programming voltage **POSV** = **3Vdd** and two transistors **N15** and **N16** are provided in the branch adapted to sustain the operative voltage **Vcp4** = **2Vdd.**

Similarly, the structures **445** and **450,** when not forming conductive paths, are be able to sustain a voltage drop equal to the programming voltage **POSV.** For the same reason described above , the structures **445** and **450** includes a number of low voltage transistors (in the example at issue, three corresponding low voltage transistors) equal to the ratio between the programming voltage **POSV** and the supply voltage **Vdd.**

Concerning the unselected word lines, each word line selector block **215** receives the corresponding first selection signal **Vp** and the corresponding second selection signal **Vp1** asserted (at the supply voltage **Vdd**). In this case, the selecting signal **OUT** is brought to the supply voltage **Vdd.** At same time, the control circuit **460** asserts the control signal **PENABLE3** and deasserts the control signals **PENABLE1** and **PENABLE2.**

The operation of the single word line selector block **215** associated with the generic unselected word line is as follows.

Since the third control signal **PENABLE3** is asserted, the transistor **N16** has the first terminal which receives the reference voltage **GND** and the control terminal which receives the supply voltage **Vdd.** In this way, the transistor **N16** is turned on (since its driving voltage is higher than its threshold voltage), thus the node **D12** reaches the reference voltage **GND.** The transistor **N15** is as well turned on (because its driving voltage is equal to the supply voltage **Vdd),** thereby bringing the node **D2** to the reference voltage **GND.**

Since the second control signal **PENABLE2** is deasserted, the transistor **N13** has the first terminal that receives the supply voltage **Vdd,** and is thus turned off (since its driving voltage is equal to zero). Thus the transistor **N14** cannot conduct any current; in this biasing condition, the voltage of the second terminal of the transistor **N14** does not interfere with the voltage of the node **D11.**

The node **D1** takes a voltage value not less than approximately the supply voltage **Vdd** minus the threshold voltage of the transistor **N14** (for example, the node voltage of the node **D11** can reach 3V - 1V = 2V).

Moreover, the transistor **N18** is turned off (because its driving voltage is equal to zero), so the transistor **N17** cannot conduct any current. Also in this case, the voltage of the second terminal of the transistor **N18** (i.e., the node **D13**) takes a value not less than approximately the supply voltage **Vdd** minus the threshold voltage of the transistor **N17** (in the example at issue, the voltage of the node **D13** reaches at most 3V-1V = 2V).

Since the first control signal **PENABLE1** is deasserted, the control terminal of the transistor **N1** receives the reference voltage **GND,** whereas the first terminal receives the supply voltage **Vdd.** In this biasing condition, the transistor **N1** is turned on, so that the voltage of the node **D3** reaches the supply voltage **Vdd.** Moreover, the transistor **N2** is turned on (since it receives at its control terminal the reference voltage **GND),** so the node **D4** is brought to the supply voltage **Vdd.** The transistor **N3** receives the reference voltage **GND** at its control terminal, and is thus turned on. In such a way, the selecting signal **OUT** reaches the supply voltage **Vdd.**

Since the control signal **PENABLE3** is asserted, the transistor **N6** has the first terminal and the control terminal that receive the reference voltage **GND.** Moreover, the transistor **N5** has the control terminal which receives the supply voltage **Vdd.** In this biasing condition, the transistor **N6** is turned off (because its driving voltage is equal to zero) and then the transistors **N5** and **N4** cannot conduct any current. Thus, the nodes **D5** and **D6** reach the voltage of the control terminal of the transistor **N5** minus its threshold voltage.

Regarding the circuital structures **445** and **450,** the transistors **N9** and **N12** are turned off, so the two structures **450** and **445** cannot conduct any current, and do not interfere with the voltage reached by the nodes **D3** and **D4,** respectively. As can be noted, during the program operation of the memory cell(s) each transistor of the single word line selector block **215** (which is coupled to a corresponding unselected word line) sustains (between its control terminal and any other terminal thereof) voltage differences at most equal to the supply voltage **Vdd.**

During an erasing operation, the selection signals **Vp** and **Vp1** are deasserted (at the reference voltage **GND**). More in detail, the control circuit **460** asserts the control signal **PENABLE1,** and deasserts the control signals **PENABLE2** and **PENABLE3.**

Since the control signal **PENABLE2** is deasserted, the first terminal of the transistor **N13** receives the supply voltage **Vdd.** Moreover, the transistors **N18** and **N17** receive at their control terminals the supply voltage **Vdd,** and are turned on. In such a way, the voltage of the node **D11** reaches the reference voltage **GND.** In this biasing condition, the transistors **N13** and **N14** are turned off (since their driving voltages are lower than their corresponding threshold voltages). Since the third control signal **PENABLE3** is deasserted, the first terminal of the transistor **N16** receives the seventh operative voltage **Vcp7** (for example, **Vcp7** = -3V). In this biasing condition, the transistor **N16** is turned on, and brings the node **D12** to -3V. The transistor **N15** is turned on, as well (because its driving voltage is higher than its threshold voltage) so the node **D2** reaches the operative voltage **Vcp7.**

The transistor **N6** has the first terminal and the gate terminal that receive the eighth operative voltage **Vcp8** (in the example at issue, **Vcp8** = -6V) and the seventh operative voltage **Vcp7** (in the example at issue, **Vcp7** = -3V), respectively, and is thus turned on. In such a way, the node **D6** reaches -6V. The transistor **N5** is turned on, as well, and brings the node **D5** to the operative voltage **Vcp8** (*i.e.*, 6V). In this biasing condition, the transistor **N4** is turned on, and the selecting signal **OUT** reaches the operative voltage **Vcp8.**

The two circuital structures **445** and **450** bring the nodes **D3** and **D4** to the reference voltage **GND** and the seventh operative voltage **Vcp7,** respectively, so the transistors **N1, N2** and **N3** are turned off and do not interfere with the voltage of the selecting signal **OUT** (since they do not conduct any current).

In particular, in the circuital structure **445,** the transistor **N9** is turned on, and brings the node **D8** to the reference voltage **GND.** In such a way, the transistor **N8** is turned on (since its driving voltage is equal to the supply voltage **Vdd**) and the voltage of the node **D7** reaches the reference voltage **GND.** The transistor **N7** is turned on as well, and brings the node **D3** to the reference voltage **GND.** Similarly to the circuital structure **445,** the circuital structure **450** forms a conductive path adapted to bring the node **D4** to the seventh operative voltage **Vcp7** (in the example at issue, to -3V). For this purpose, the first terminal of the transistor **N12** receives the seventh operative voltage **Vcp7,** and the transistor **N12** is turned on (in fact, the transistor **N12** has the driving voltage equal to the supply voltage **Vdd**). The voltage of the node **D10** can reach the operative voltage **Vcp7,** thereby the transistor **N11** is turned on. In this way, the node **D9** reaches the operative voltage **Vcp7** as well, and turns the transistor **N10** on, which brings the node **D4** to the operative voltage **Vcp7.**

As can be noted, during the erasing operation of the memory cell(s) (which are coupled to the selected word lines of the sector) each transistor of the single word line selector block **215** sustains (between its control terminal and any other terminal thereof) voltage differences at most equal to the supply voltage **Vdd.** In other words, the single word line selector block **215** can be implemented with low voltage transistors only.

As described above, during the erasing operation the branch including the transistors **N13** and **N14** and the branch including the transistors **N1, N2** and **N3** are not conductive paths, and do not interfere with the voltage which is reached by the node **D2** and **D60,** respectively. In this biasing condition, the branch including the transistors **N13** and **N14** sustains a voltage drop equal to the difference between the supply voltage **Vdd** and the operative voltage **Vcp7** (in the example at issue, 3V - (-3V) = 6V = **2Vdd**) without breaking of the low voltage transistors. The branch including the transistors **N1, N2** and **N3** sustains a voltage drop equal to the difference between the supply voltage **Vdd** and the operative voltage **Vcp8** (in the example at issue, equal to 3V- (-6V) = 9V = **3Vdd).** As can be noted, also in this case each branch includes a number of transistors equal to the voltage drop to be sustained divided by the supply voltage **Vdd,** in order to avoid undesired breaking of low voltage transistors thereof.

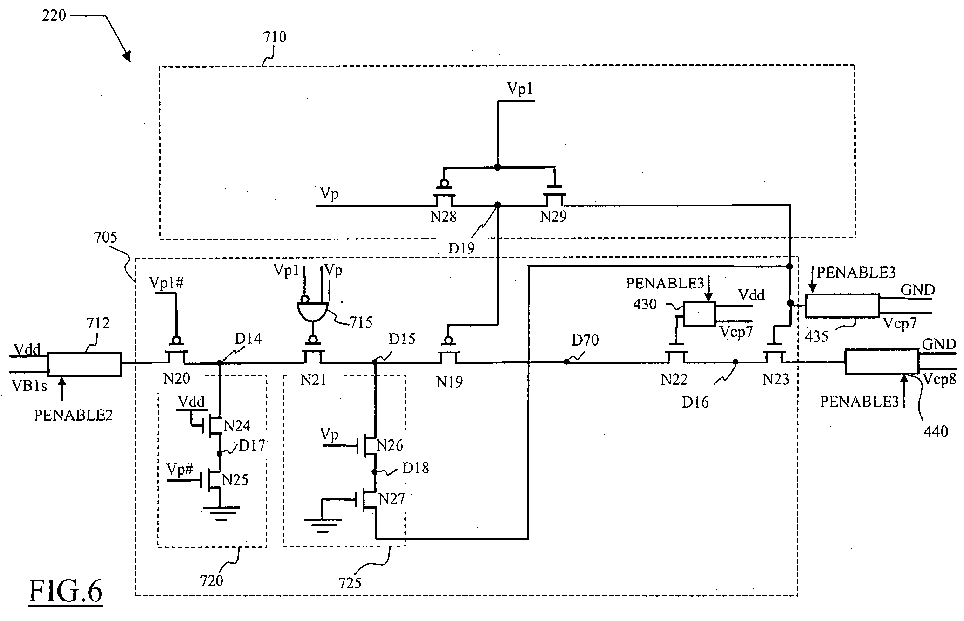

Moving to **Figure 6****,** an exemplary implementation of a generic first biasing block **220** is schematically shown. The first biasing block **220** includes a first circuital block **705** and a second circuital block **710.**

The first circuital block **705** includes a first circuit branch with three p-channel MOSFETs **N19, N20** and **N21** connected in series, and a second circuit branch with two further series-connected n-channel MOSFETs **N22** and **N23,** the first and second circuit branches being both connected to a common node **D70.** In particular, the transistor **N20** has a control terminal which is adapted to receive the complemented first selection signal **Vp1** and a first terminal which is connected to the output terminal of an eleventh voltage switch **712.** The voltage switch **712** has two input terminals, respectively adapted to receive the first biasing voltage **VB1s** = 6V and the supply voltage **Vdd.** The voltage switch **712** is controlled by the second control signal **PENABLE2,** and, according to the assertion state thereof, one of the switch input terminals is connected to the switch output terminal, so that the first terminal of the transistor **N20** can be alternatively biased at the first biasing voltage **VB1s** or at the supply voltage **Vdd.**

A second terminal (denoted as node **D14**) of the transistor **N20** is connected to a first terminal of the transistor **N21,** which has a control terminal connected to an output terminal of a two-input AND gate **715.** The AND gate **715** receives the first selection signal **Vp** and the complemented second selection signal **Vp1.** The transistor **N21** has a second terminal (denoted as node **D15**) connected to a first terminal of the transistor **N19.**

The second terminal (corresponding to the node **D70**) of the transistor **N19** is connected to a second terminal of the transistor **N22** and provides the first control signal **VB1.**

A first terminal of the transistor **N22** (denoted as node **D16**) is connected to a second terminal of the transistor **N23** which has its control and first terminals coupled to the voltage switches **435** and **440,** respectively. Moreover, the control terminal of the transistor **N22** is connected to a switch output terminal of the voltage switch **430.**

The first circuital block **705** further includes two circuital structures **720** and **725.**

In detail, the circuital structure **720** includes two n-channel MOSFETs **N24** and **N25** which are connected in series. In particular, the transistor **N24** has a second terminal connected to a second terminal of the transistor **N20** and a first terminal (denoted as node **D17**) connected to a second terminal of the transistor **N25.** The transistor **N24** has a control terminal which receives the supply voltage **Vdd.** The transistor **N25** has a first terminal which is adapted to remain at ground whereas at its control terminal it receives the complemented first selection signal **Vp**#.

The circuital structure **725** includes two series-connected n-channel MOSFETs **N26** and **N27.** In particular, the transistor **N26** has a second terminal connected to the node **D15** and a first terminal (denoted as node **D18**) connected to a second terminal of the transistor **N27.** The transistor **N26** has a control terminal which is adapted to receive the first selection signal **Vp.** The transistor **N27** has a first terminal which is connected to the output terminal of the fifth voltage switch **435** and a control terminal which receives the reference voltage **GND.**

The second circuital block **710** includes a first circuit branch with one p-channel MOSFET **N28,** and a second circuit branch with one further n-channel MOSFET **N29,** the first and second circuit branches being both connected to a common node **D19.** In particular, the transistor **N28** has a first terminal which is adapted to receive the first selection signal **Vp** and a second terminal which is connected to a second terminal (*i.e*., the node **D19**) of the transistor **N29.** Moreover, a control terminal of the transistor **N28** is connected to a control terminal of the transistor **N29** and receives the second selection signal **Vp1.** A first terminal of the transistor **N29** is connected to the output terminal of the voltage switch **435.**

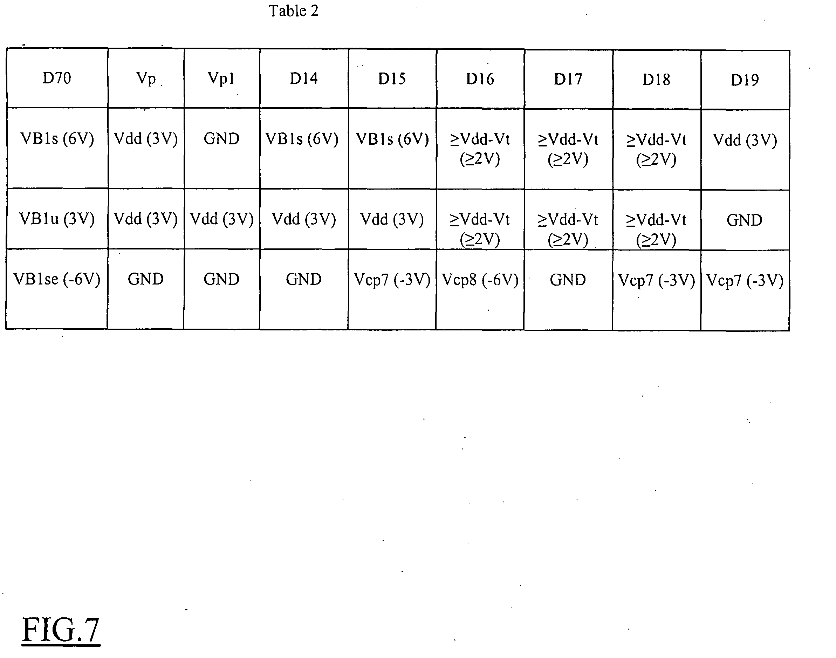

Referring now to **Figure 7** in combination with **Figure 6****,** a **Table 2** is shown, illustrating the voltages of the nodes of the first biasing block **220** according to the operation to be performed on the memory device **100.** In particular, according to the assertion state of the selection signals **Vp** and **Vp1,** the first biasing block **220** provides the first control signal **VB1.**

During a program operation, the selection signal **Vp** is asserted (at the supply voltage **Vdd**); when the selection signal **Vp1** is de-asserted (at the reference voltage **GND),** the corresponding word line is selected for the program operation of the memory cell coupled thereto. More in detail, the control circuit **460** asserts the control signals **PENABLE2** and **PENABLE3** and causes the node **D70** to reach the first control voltage **VB1s** (for example, **VB1s** = 6V). In other words, by means a proper biasing of the first biasing block **220,** the node **D70** can reach the first control voltage **VB1s** (for example, **VB1s** = 6V). In fact, as described above referring to **Figure 4****,** during a program operation, for selecting the word line to which the memory cell(s) to be programmed belong(s), the first biasing block **220s** provides the first control signal **VB1** at the voltage **VB1s.**

The first biasing block **220** operates as follows.

The transistor **N28** is turned on (because its driving voltage is equal to the supply voltage **Vdd**) and causes the node **D19** to reach the supply voltage **Vdd.**

Since the third control signal **PENABLE3** is asserted, the first terminal of the transistor **N29** receives the reference voltage **GND.** In such a way, the transistor **N29** is turned off (since its driving voltage is equal to zero).

Moreover, since the second control signal **PENABLE2** is asserted, the first terminal of the transistor **N20** receives the voltage **VB1s** (for example, **VB1s** = 6V) and is turned on (since its driving voltage is equal to the supply voltage **Vdd).** In such a way, the voltage of the node **D14** reaches 6V. Moreover, when the second control signal **PENABLE2** is asserted, the control terminal of the transistor **N21** receives the supply voltage **Vdd,** which turns on the transistor **N21,** bringing the node **D15** to the voltage **VB1s.** In this biasing condition, the transistor **N19** is turned on, so the node **D70** can reach the first control voltage **VB1s.**

Since the third control signal **PENABLE3** is asserted, the control and first terminals of the transistor **N23** receive the reference voltage **GND,** and the transistor is turned off. Thus, the transistor **N22** cannot conduct any current, and the voltage value at the node **D16** remains essentially at the supply voltage **Vdd** minus the threshold voltage of the transistor **N22.**

During the program operation, the two structures **720** and **725** do not form conductive paths, so they do not interfere with the voltages of the nodes **D14** and **D15,** respectively. In detail, in the structure **720** the transistor **N25** is turned off (because its driving voltage is equal to zero). Thus, the transistor **N24** cannot conduct any current and the voltage at the node **D17** remains essentially at the supply voltage Vdd minus the threshold voltage of the transistor **N24.** Likewise, in the structure **725** the transistor **N27** is turned off, and the node **D18** remains at the supply voltage **Vdd** (which is the voltage of the control terminal of the transistor **N26**) minus the threshold voltage of the transistor **N26.**

As can be noted, during the program operation of the memory cell(s) corresponding to the selected word line, each transistor of the first biasing block **220** sustains at its control and first/second terminals at most the supply voltage **Vdd.**

Moreover, the circuital branch including the transistors **N22** and **N23** and the circuital branch including the transistor **N29** respectively have a number of low voltage transistors equal to the ratio between the voltage drop to be sustained when each branch is not conductive divided by the supply voltage **Vdd.**

Similarly, the circuital structures **720** and **725** have a number of low voltage transistors equal to the ratio between the voltage drop to be sustained when each circuital structure is not conductive divided by the supply voltage **Vdd** (in the example at issue, the pairs of transistors **N24-N25** and **N26-N27,** respectively).

Concerning the unselected word lines, each first biasing block **220** receives the corresponding first selection signal **Vp** and the corresponding second selection signal **Vp1** asserted (at the supply voltage **Vdd**). In this case, the voltage of the node **D70** reaches the first control voltage **VB1u** (for example, **VB1u** = 3V). In other words, as described above referring to **Figure 4****,** during a program operation the first biasing block **220u** provides the first control signal **VB1** at the voltage **VB1u.** In particular, according to the assertion state of the first and second selecting signals **Vp** and **Vp1,** the control circuit **460** de-asserts the second control signal **PENABLE2** (at the reference voltage **GND**) and asserts the third control signal **PENABLE3** (at the supply voltage **Vdd**).

Since the second control signal **PENABLE2** is deasserted, the first terminal of the transistor **N20** receives the supply voltage **Vdd.** The control terminal of the transistor **N21** receives the reference voltage **GND.** Moreover, since the third control signal **PENABLE3** is asserted, the first terminal and the control terminal of the transistor **N23** receive the reference voltage **GND,** whereas the control terminal of the transistor **N22** receives the supply voltage **Vdd.**

When the third control signal **PENABLE3** is asserted, the transistor **N29** has the first terminal receiving the reference voltage **GND.** In this biasing condition, the transistor **N29** is turned on (since its driving voltage is equal to the supply voltage **Vdd**) and causes the node **D19** to reach the reference voltage **GND.**

Moreover, the driving voltage of the transistor **N28** is equal to zero, and the transistor is turned off.

The transistor **N20** is turned on (since its driving voltage is equal to the supply voltage **Vdd),** thereby bringing the node **D14** to the supply voltage **Vdd.** In this biasing condition, the transistor **N21** is turned on and causes the node **D15** to reach the supply voltage **Vdd.** The transistor **N19** is thus turned on, so the voltage of the node **D70** reaches the supply voltage **Vdd.** In other words, the first control signal **VB1** reaches the voltage **Vdd** (in the example at issue, **VB1u** = **Vdd** = 3V).

The transistor **N23** is turned off (because its driving voltage is equal to zero) and thus transistor **N22** cannot conduct any current. In such a way, the voltage of the node **D16** takes a value to not less than the supply voltage **Vdd** (*i.e.,* the voltage of the control terminal of the transistor **N22**) minus the threshold voltage of the transistor **N22.**

Also in this case, the circuital structures **720** and **725** do not form conductive paths, so they do not interfere with the voltages of the nodes **D14** and **D15,** respectively. Moreover, the nodes **D17** and **D18** reach the same voltages (as shown in **Table 2**) of the preceding case in which the first biasing block **220** biases the selected word line.

During an erase operation, the first and second selection signals **Vp** and **Vp1** are deasserted (at the reference voltage **GND**). More in detail, the control circuit **460** deasserts the control signals **PENABLE2** and **PENABLE3,** thereby allowing to select each word line of the sector **105.**

Since the control signal **PENABLE2** is deasserted, the first terminal of the transistor **N20** receives the supply voltage **Vdd.** The control terminal of the transistor **N21** receives the reference voltage **GND.**

Moreover, since the third control signal **PENABLE3** is deasserted, the transistor **N23** has the first terminal and the control terminal which receive the operative voltages **Vcp7** and **Vcp8,** respectively. In the example at issue, **Vcp7** = - 3V and **Vcp8** = -6V. The transistor **N22** has the control terminal which receives the operative voltage **Vcp7.**

In this biasing condition, the transistor **N29** is turned on (since its first terminal receives -3V) thus causing the node **D19** to reach the operative voltage **Vcp7** (in the example at issue, -3V). In such a way, the transistor **N28** is turned off.

The transistor **N23** is turned on (because its driving voltage is equal to the supply voltage **Vdd**) and causes the node **D16** to reach the operative voltage **Vcp8** (in the example at issue, -6V). In such a way, the transistor **N22** is turned on and brings the node **D70** to the operative voltage **Vcp8.** In other words, the voltage of the node **D70** (i.e., the voltage **VB1se**) is equal to the operative voltage **Vcp8.**

During the erase operation the circuital structures **720** and **725** forms conductive paths adapted to bring the nodes **D14** and **D15** to the reference voltage **GND** and to the operative voltage **Vcp7,** respectively.

In particular, in the circuital structure **720** the transistor **N25** is turned on (since its driving voltage is equal to the supply voltage **Vdd)** and causes the node **D17** to reach the reference voltage **GND.** The transistor **N24** is turned on as well, and causes the node **D14** to reach the reference voltage **GND.**

Likewise, in the circuital structure **725,** the transistor **N27** is turned on so that the node **D18** can reach the operative voltage Vcp7 (in the example at issue, -3V). In such a way, the transistor **N26** is turned on and brings the node **D15** to the operative voltage **Vcp7.**

In such a way, the transistor **N19** is turned off and not interfere with the voltage of the node **D70.**

As can be noted, during the erasing operation of the memory cell(s) (coupled to the selected word lines), each transistor of the first biasing block **220** sustain across its control and first/second terminals at most the supply voltage **Vdd.** In other words, the first biasing block **220** can be implemented with low voltage transistors only.

Moreover, the circuital branch including the transistors **N19, N20** and **N21** and the circuital branch including the transistor **N28** respectively have a number of low voltage transistors equal to the ratio between the voltage drop to be sustained when each branch is not conductive divided by the supply voltage **Vdd.**

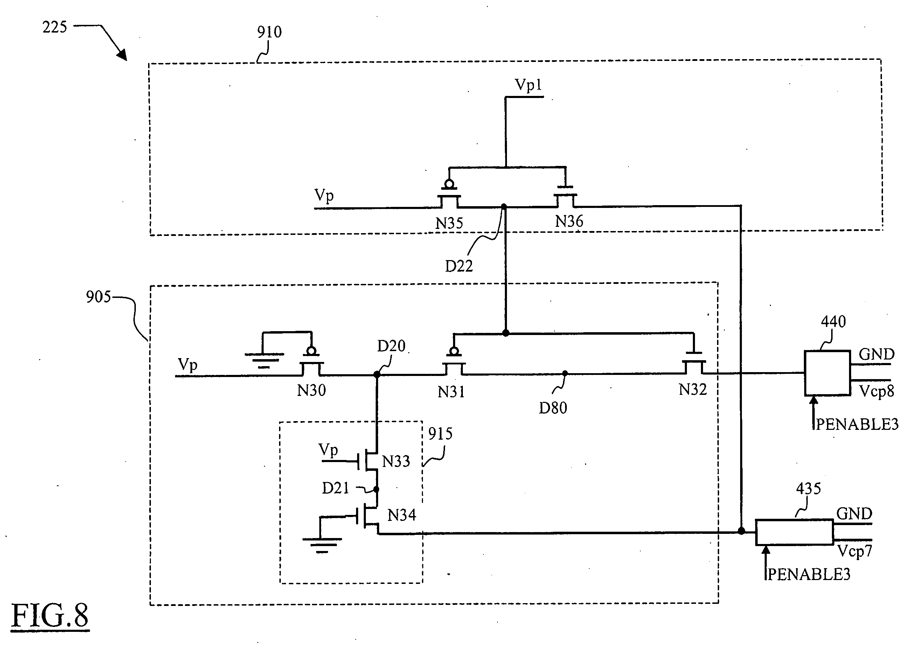

Referring to **Figure 8****,** an exemplary implementation of a generic second biasing block **225** is schematically shown. The second biasing block **225** includes a first circuital block **905** and a second circuital block **910.**

The first circuital block **905** includes a first circuit branch with one n-channel MOSFET **N32,** and a second circuit branch with two series-connected p-channel MOSFETs **N30** and **N31,** the first and second circuit branches being both connected to a common node **D80.**

In particular, the transistor **N30** has a control terminal adapted to receive the reference voltage **GND** and a first terminal receiving the first selection signal **Vp.** A second terminal (denoted as node **D20**) of the transistor **N30** is connected to a first terminal of the transistor **N31** which has a control terminal connected to a control terminal of the transistor **N32.**

A second terminal of the transistor **N31** (corresponding to the node **D80)** which, during the operation of the second biasing block **225,** provides the second control signal **VB2,** is connected to a second terminal of the transistor **N32.** The voltage switch **440** selectively connects one of its two input terminals to a first terminal of the transistor **N32** according to the operation to be performed on the memory device.

The first circuital block **905** further includes a circuital structure **915.** In detail, the circuital structure **915** includes two n-channel MOSFETs **N33** and **N34** which are connected in series. In particular, the transistor **N33** has a second terminal connected to the second terminal of the transistor **N30** and a first terminal (denoted as node **D21**) connected to a second terminal of the transistor **N34.** The transistor **N34** has a control terminal which receives the reference voltage **GND** and a first terminal coupled to the output of the voltage switch **435.**

The second circuital block **910** includes a p-channel MOSFET **N35** connected in series to a n-channel MOSFET **N36.** In particular, the second circuital block **910** has a same circuital structure as the second circuital block **710** (in an alternative embodiment of the invention, the second biasing block **225** may consists in the first circuital block **905** directly connected to the second circuital block **710** of the first biasing block **220**). In detail, the transistor **N35** has a second terminal which is connected to a second terminal (denoted as node **D22**) of the transistor **N36** and also connected to the control terminals of the transistors **N31** and **N32.**

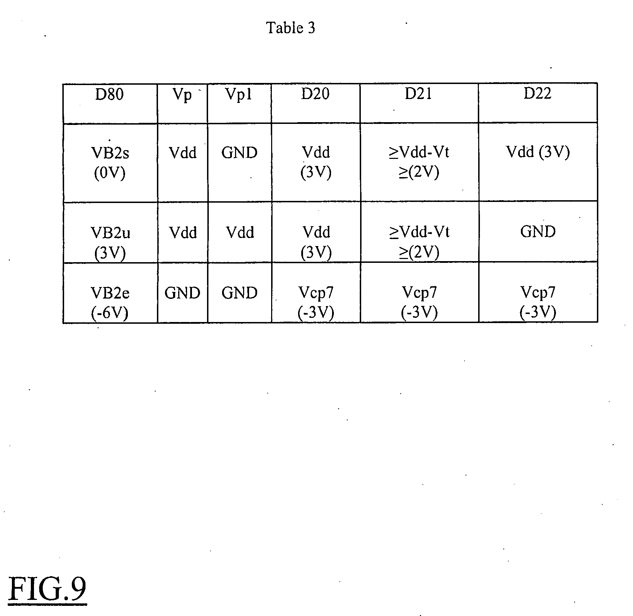

Referring now jointly to **Figures 8** and **9****,** a **Table 3** is shown in **Figure 9****,** reporting exemplary voltages of the most significant nodes of the second biasing block **225** depending on the operation to be performed on the memory device.

In particular, according to the assertion state of the first and second selection signals **Vp** and **Vp1,** the second biasing block **225** provides the second control signal **VB2.**

During a program operation, the selection signal **Vp** is asserted (at the supply voltage **Vdd**); when the selection signal **Vp1** is de-asserted (at the reference voltage **GND),** the corresponding word line is selected for the program operation of the memory cell(s) coupled thereto. More in detail, the control circuit **460** asserts the control signals **PENABLE2** and **PENABLE3** and causes the node **D80** to reach the voltage **VB2s** (for example, **VB2s** = 0V). In other words, by means a proper biasing of the first biasing block **225,** the node **D80** can reach the voltage **VB2s.** In fact, as described above referring to **Figure 4****,** during a program operation, for selecting the word line to which the memory cell(s) to be programmed belong(s), the first biasing block **225s** provides the second control signal **VB2** at the voltage **VB2s.**

In this biasing condition, as described above referring to the second circuital block **710,** the node **D22** of the second circuital block **910** reaches the supply voltage **Vdd.** Moreover, since the third control signal **PENABLE3** is asserted, the transistor **N32** has the first terminal which receives the reference voltage **GND** by the voltage switch **440.**

The transistor **N32** is thus turned on (because its driving voltage is higher than its threshold voltage) and causes the node **D80** to reach the reference voltage **GND** (corresponding to the desired voltage for the second control signal **VB2**).

The transistor **N30** is turned on (since its driving voltage is equal to the supply voltage **Vdd**) and brings the node **D20** to the supply voltage **Vdd.** In such a way the transistor **N31** is turned off.

In this biasing condition, the circuital structure **915** forms a not conductive path. In detail, the transistor **N34** has the first terminal which receives the reference voltage **GND** and is thus turned off. The transistor **N33** (being connected in series to the transistor **N34)** cannot conduct any current. Thus, the voltage of the node **D21** takes a value not less than the supply voltage **Vdd** minus the threshold voltage of the transistor **N33** (for example, as shown in **Table 3,** the voltage of the node **D21** reaches at most 3V - 1V = 2V).

Concerning the unselected word lines, each second biasing block **225** receives the corresponding first selection signal **Vp** and the corresponding second selection signal **Vp1** asserted (at the supply voltage **Vdd**). In this case, the voltage of the node **D80** reaches the voltage **VB2u** (for example, **VB2u** = 3V). In other words, as described above referring to **Figure 4****,** during a program operation the second biasing block **225u** corresponding to the unselected word line provides the second control signal **VB2** at the voltage **VB2u.**

The control circuit **460** asserts the control signal **PENABLE3** and deasserts the control signal **PENABLE2.** The operation of the second biasing block **225u** associated with the generic unselected word line is as follows.

As described above referring to the second circuital block **710,** the node **D22** of the second circuital block **910** reaches the reference voltage **GND,** which is fed to the control terminals of the transistors **N31** and **N32.** Moreover, since the third control signal **PENABLE3** is asserted, the transistors **N32** and **N34** have the corresponding first terminal which receives the reference voltage **GND.**

The transistor **N30** is turned on (because its driving voltage is higher than its threshold voltage) and causes the node **D20** to reach the supply voltage **Vdd.** The transistor **N31** is turned on, so that the voltage of the node **D80** reaches the supply voltage **Vdd** (*i.e.*, **VB2u** = 3V). In such a way, the transistor **N32** is turned off.

Also in this case, the circuital structure **915** forms a not conductive path, and the voltage of the node **D21** reaches a value to not less than the supply voltage **Vdd** minus the threshold voltage of the transistor **N33.**

As can be noted, during the programming operation of the memory cell(s), each transistor of the second biasing block **225** sustains across its control and first/second terminals at most the supply voltage **Vdd.**

Moreover, the circuital branch including the transistor **N32** has only one low voltage transistor, since the ratio between the voltage drop to be sustained when said branch is not conductive divided by the supply voltage **Vdd** is equal to one.

Similarly, the circuital structure **915** has a number of low voltage transistors equal to the ratio between the voltage drop to be sustained when said circuital structure is not conductive divided by supply voltage **Vdd** (in the example at issue, the pair of transistors **N33-N34**).

During an erase operation, the first and second selection signals **Vp** and **Vp1** are deasserted (at the reference voltage **GND**). More in detail, the control circuit **460** deasserts the control signals **PENABLE2** and **PENABLE3** thereby allowing to select all the word lines of the sector to be erased.

Since the control signal **PENABLE3** is deasserted, the transistors **N32** and **N34** have the corresponding first terminal which receives the operative voltages **Vcp8** and **Vcp7,** respectively. In the example at issue, **Vcp7** = -3V and **Vcp8** = -6V. Moreover, the transistors **N32** and **N31** have the control terminal which receives the operative voltage **Vcp7.**

In this biasing condition, the transistor **N32** is turned on and causes the node **D80** to reach the operative voltage **Vcp8.** In the example at issue, **Vcp8** = -6V (i.e., **VB2se** = -6V).

During the erase operation the circuital structure **915** forms a conductive path adapted to bring the node **D20** to the operative voltage **Vcp7.** In particular, in the circuital structure **915** the transistor **N34** is turned on (since its driving voltage is equal to the supply voltage **Vdd**) and causes the node **D21** to reach the operative voltage **Vcp7.** In this biasing condition, the transistor **N33** is turned on so that the node **D20** can reach the operative voltage **Vcp7.**

In such a way, the transistors **N30** and **N31** are turned off, and do not interfere with the voltage (i.e., **VB2se** = -6V) of the node **D80.**

As can be noted, during the operation of the second biasing block **225** each transistor thereof sustains (between its control terminal and any other terminal thereof) voltage differences at most equal to the supply voltage **Vdd.** Thus, the second biasing block **225** can be implemented with low voltage transistors only. Also in this case, the circuital branch including the transistors **N30** and **N31** includes a number of low voltage transistors equal to the ratio between the voltage drop to be sustained when said branch is not conductive and the supply voltage **Vdd.**

According to the present invention, the possibility of using only low voltage transistors for implementing the row selector of a memory device is contemplated, even in the case the voltage values to be handled are higher (in absolute value) than the supply voltage **Vdd,** or lower than the reference voltage **GND.** As described above, a low voltage transistor is a device designed in such a way to guarantee the capability of sustaining, at least between a pair of its terminals, and particularly at least between the control terminal and another one of its terminals, voltage differences up-limited by the supply voltage **Vdd.**

The possibility of including only low voltage devices in the row selector allows to significantly simplify the manufacturing process of the memory device.

The present invention provides a row selector which by adopting a particular circuital topology avoids the use of HV transistors. More in particular, according to the preferred embodiment of the present invention, such a result is achieved by providing, in some circuital structures of the row selector, a number of series-connected LV transistors equal to the ratio of the voltage drop (in absolute value) that the generic circuital structure has to withstand when it is not conductive, to the maximum voltage difference that a single LV transistor can sustain across its terminals, particularly across its control and source/gate or bulk terminal (*i.e.*, Vdd = 3 Volts in case of LV transistors).

In other words, the number of low voltage transistors connected in series in order to form the circuital structures of the row selector depends to the maximum voltage drop that each circuital structure is able to sustain when forms a not conductive path.

For example, the circuital structure **235u** of the intermediate circuit **210** when form a not conductive path sustains a maximum voltage drop equal to the programming voltage **POSV.** In the preferred embodiment of the invention, the programming voltage **POSV** is equal to an integer multiple of the supply voltage **Vdd** (for example **POSV** = **3Vdd**), so that the circuital structures **235u** can be implemented trough a number of low voltage transistors equal to the integer multiple (in the example at issue, the three transistors **M4u, M5u** and **M6u**).

Naturally, in order to satisfy local and specific requirements, a person skilled in the art may apply to the solution described above many modifications and alterations. Particularly, although the present invention has been described with a certain degree of particularity with reference to preferred embodiment(s) thereof, it should be understood that various omissions, substitutions and changes in the form and details as well as other embodiments are possible; moreover, it is expressly intended that specific elements and/or method steps described in connection with any disclosed embodiment of the invention may be incorporated in any other embodiment as a general matter of design choice.

It should be apparent that the numerical examples of the different voltages described above are merely illustrative and must not to be interpreted in a limitative manner.

In any case, the use of other types of transistors, (for example, bipolar junction transistors) is within the scope of the invention.

Similar considerations apply if the memory device has a different structure or includes equivalent components.

Likewise, it is possible to use the proposed solution for biasing the selected bit lines during the operations performed on the memory device.

## Claims

1. A row selector for a semiconductor memory including a plurality of memory cells coupled to a corresponding plurality of word lines, the row selector comprising, for each word line:
- a first biasing circuit path (230; 215; 260) adapted to bias the corresponding word line to a programming voltage (POSV) when said corresponding word line is selected for selectively performing a program operation on at least one memory cell coupled to the corresponding word line, the first biasing circuit path comprising programming voltage provisioning means (215) adapted to provide the programming voltage (POSV);
- a second biasing circuit path (235) which is adapted to receive, from program-inhibit voltage provisioning means (250), a program inhibit voltage (GND), and to provide to the corresponding word line said program inhibit voltage (GND) when the word line is unselected during the program operation;
- first biasing means (220, 225) for driving the second biasing circuit path (235) in order to control a conduction state thereof;wherein:
- said first biasing circuit path (230,215,260) includes a first transistor (M2) controlled to be electrically conductive when the corresponding word line is selected, and to be electrically non-conductive when the corresponding word line is unselected;
- said first biasing means (220,225) controls the second biasing circuit path to be conductive when, during the program operation, the corresponding word line is unselected,
- said second biasing circuit path (235) includes a plurality of series-connected transistors, the number of transistors in said plurality being at least equal to the smallest integer not less than an absolute value of a ratio between a voltage equal to the difference between the programming voltage (POSV) and the program-inhibit voltage (GND) to a predetermined maximum voltage;
**characterised in that**:
said predetermined maximum voltage is the maximum voltage which a transistor of said series connected transistors can sustain before it breaks down.

2. The row selector according to claim 1, wherein the second biasing circuit path is adapted to receive from erase voltage provisioning means **(250)** an erasing voltage **(NEGEV)** and to provide to the corresponding word line said erasing voltage during an erase operation performed on at the least two word lines of said plurality, said first biasing means controlling the second biasing circuit path so as to be conductive when performing the erase operation.

3. The row selector according to claim 1 or 2, wherein each transistor of said plurality is adapted to guarantee the capability of sustaining voltage differences across at least a control terminal and another terminal thereof that are up-limited in absolute value by a supply voltage **(Vdd)** of the semiconductor memory.

4. The row selector according to claim 2 or 3, wherein said plurality of transistors includes a second transistor **(M6)** controlled by said first biasing means to be conductive during the erase operation and when the corresponding word line is unselected.

5. The row selector according to claim 2, 3 or 4, wherein the first transistor is adapted to guarantee the capability of sustaining, at least across a control terminal and another terminal thereof, voltage differences that are up-limited in absolute value by the supply voltage.

6. The row selector according to any claim from 1 to 5, wherein the first biasing circuit path is further adapted to bias the corresponding word line to a reading voltage **(Vcp1)** when said corresponding word line is selected for selectively performing a read operation on at least one memory cell coupled to the corresponding word line, the first biasing circuit path comprising reading voltage provisioning means **(260)** adapted to provide the reading voltage.

7. The row selector of claim 6, wherein the first biasing circuit path further includes a third transistor **(M3)** adapted to convey the programming voltage from said programming voltage provisioning means **(215)** when said corresponding word line is selected for selectively performing the program operation, and to convey the reading voltage from said reading voltage provisioning means **(260)** when said corresponding word line is selected for selectively performing the reading operation, said third transistor **(M3)** being adapted to guarantee the capability of sustaining at least across a control terminal and another terminal thereof voltage differences that are up-limited in absolute value by the supply voltage of the semiconductor memory.

8. The row selector according to claim 6 or 7 as depending on claim 2, further including second biasing means **(240, SW4, SW3)** for controlling the third transistor in such a way as to be conductive when the corresponding word line is selected for selectively performing the program operation or alternatively for selectively performing the read operation, and for controlling the third transistor to be not conductive during the erase operation.

9. The row selector according to claim 8, wherein the first circuit path includes a fourth transistor **(M1)** coupling the second biasing means **(240, SW4, SW3)** to a control terminal of the third transistor **(M3),** the fourth transistor being adapted to guarantee the capability of sustaining, at least across a control terminal and another terminal thereof, voltage differences that are up-limited in absolute value by the supply voltage.

10. The row selector according to claim 9, wherein the first biasing means include a first biasing block **(220)** which is adapted to provide a first control signal **(VB1)** controlling the first transistor, said first control signal being at a first selecting control voltage **(VB1s)** adapted to cause the first transistor to be conductive when the corresponding word line is selected for selectively performing the program operation, said first control signal being at a first unselecting control voltage **(VB1u)** when the corresponding word line is unselected during the program operation and at a first erasing control voltage **(VB1se)** during the erase operation, the first unselecting control voltage and the first erasing control voltage being adapted to cause the first transistor to be not conductive.

11. The row selector according to claim 9 or 10, wherein the first biasing means include a second biasing block **(225)** which is adapted to provide a second control signal **(VB2)** controlling the second transistor, said second control signal being at a second selecting control voltage **(VB2s)** adapted to cause the second transistor to be not conductive when the corresponding word line is selected for selectively performing the program operation, said second control signal being at a second unselecting control voltage **(VB2u)** when the corresponding word line is unselected during the program operation and at a second erasing control voltage **(VB2se)** during the erase operation, the second unselecting control voltage and the second erasing control voltage being adapted to cause the second transistor to be conductive.

12. The row selector according to claim 11, wherein at least one of the programming voltage provisioning means, the first biasing block and the second biasing block includes a corresponding first circuit branch **(N4-N6; N22-N23; N32)** and second circuit branch **(N1-N3; N19-N21; N30-N31)** which are connected to a corresponding output circuit node **(D60, D70, D80),** the first circuit branch and the second circuit branch being adapted to receive at a corresponding input terminal thereof a corresponding first and second operative input signal, said output circuit node being adapted to provide a corresponding output signal depending on the first or second operative input signals, the first circuit branch and the second circuit branch including a corresponding first and second plurality of series-connected transistors, a corresponding number of transistors in said first plurality being at least equal to the smallest integer not less than an absolute value of a ratio between a first drop voltage across the first circuit branch when said first circuit branch is not conductive and the predetermined maximum voltage, a corresponding number of transistors in said second plurality being at least equal to the smallest integer not less than an absolute value of a ratio between a second drop voltage across the second circuit branch when said second circuit branch is not conductive and the predetermined maximum voltage.

13. The row selector according to claim 6 to 12, wherein said programming voltage is first multiple of the supply voltage, said reading voltage is a second multiple of the supply voltage, said erasing voltage is a third multiple of the supply voltage, the programming voltage and the reading voltage having a first sign, the erasing voltage having a second sign, the second sign being opposed to the first sign, at least one of the first multiple, the second multiple and the third multiple being higher in absolute value than the supply voltage.

14. The row selector according to claim from 9 to 13, wherein the first, second, third, fourth transistors are MOSFETs.

## Patentansprüche

1. Reihenselektor für einen Halbleiterspeicher mit einer Mehrzahl von Speicherzellen, die mit einer entsprechenden Mehrzahl von Wortleitungen gekoppelt sind, wobei der Reihenselektor für jede Wortleitung Folgendes umfasst:
- eine erste Vorspannungsschaltungsbahn (230; 215; 260) zum Vorspannen der entsprechenden Wortleitung auf eine Programmierspannung (POSV), wenn die genannte entsprechende Wortleitung zum selektiven Ausführen einer Programmoperation an wenigstens einer Speicherzelle selektiert ist, die mit der entsprechenden Wortleitung gekoppelt ist, wobei die erste Vorspannungsschaltungsbahn ein Piogrammierspamiungscrzcugwigsmittel (215) zum Erzeugen der Programmierspannung (POSV) umfasst;
- eine zweite Vorspannungsschaltungsbahn (235), um von einem Programmsperrspannungserzeugungsmittel (250) eine Programmsperrspannung (GND) zu empfangen und die genannte Programmsperrspannung (GND) an die entsprechende Wortleitung anzulegen, wenn die Wortleitung während der Programmoperation unselektiert ist;
- ein erstes Vorspannungsmittel (220, 225) zum Ansteuern der zweiten Vorspannungsschaltungsbahn (23S), um einen Leitungszustand davon zu steuern; wobei:
- die genannte erste Vorspannungsschaltungsbahn (230, 215, 260) einen ersten Transistor (M2) aufweist, der so gesteuert wird, dass er elektrisch leitend ist, wenn die entsprechende Wortleitung selektiert ist, und elektrisch nichtleitend ist, wenn die entsprechende Wortleitung unselektiert ist;
- wobei das genannte erste Vorspannungsmittel (220, 225) die zweite Vorspannungsschaltungsbahn so steuert, dass sie leitend ist, wenn die entsprechende Wortleitung während der Programmoperation unselektiert ist,
- wobei die genannte zweite Vorspannungsschaltungsbahn (235) eine Mehrzahl von in Serie geschalteten Transistoren aufweist, wobei die Zahl der Transistoren in der genannten Mehrzahl wenigstens gleich der kleinsten ganzen Zahl ist, die nicht kleiner ist als ein absoluter Wert eines Verhältnisses zwischen einer Spannung, die gleich der Differenz zwischen der Programmierspannung (POSV) und der Programnsperrspannung (GND) ist, und einer vorbestimmten Höchstspannung;
- **dadurch gekennzeichnet, dass**:
die genannte vorbestimmte Höchstspannung die höchste Spannung ist, die ein Transistor der genannten in Serie geschalteten Transistoren aushalten kann, bevor er zusammenbricht.

2. Reihenselektor nach Anspruch 1, wobei die zweite Vorspannungsschattungsbahn die Aufgabe hat, eine Löschspannung (NEGV) von einem Löschspannungserzeugungsmittel (250) zu empfangen und die genannte Löschspannung an die entsprechende Wortleitung während einer Löschoperation anzulegen, die an den wenigstens zwei Wortleitungen der genannten Mehrzahl ausgeführt wird, wobei das genannte erste Vorspannungsmittel die zweite Vorspannungsschaltungsbahn so steuert, dass sie leitend ist, wenn sie die Löschoperation ausführt.

3. Reihenselektor nach Anspruch 1 oder 2, wobei jeder Transistor der genannten Mehrzahl die Aufgabe hat, die Fähigkeit des Aushaltens von Spannungsdiffcrenzen über wenigstens einen Steueranschluss und einen anderen Anschluss davon zu garantieren, deren absolute Werte durch eine Speisespannung (Vdd) des Halbleiterspeichers nach oben begrenzt werden.

4. Reihenselektor nach Anspruch 2 oder 3, wobei die genannte Mehrzahl von Transistoren einen zweiten Transistor (M6) aufweist, der von dem genannten ersten Vorspannungsmittel so gesteuert wird, dass er während der Löschoperation sowie dann leitend ist, wenn die entsprechende Wortleitung unselektiert ist.

5. Reihenselektor nach Anspruch 2, 3 oder 4, wobei der erste Transistor die Aufgabe hat, die Fähigkeit des Aushaltens von Spannungsdifferenzen, deren absolute Werte durch die Speisespannung nach oben begrenzt werden, wenigstens über einen Steueranschluss und einen anderen Anschluss davon zu garantieren.

6. Reihenselektor nach einem der Ansprüche 1 bis 5, wobei die erste Vorspannungsschaltungsbahn ferner die Aufgabe hat, die entsprechende Wortleitung auf eine Lesespannung (Vcp1) vorzuspannen, wenn die genannte entsprechende Wortleitung zum selektiven Ausführen einer Leseoperation an wenigstens einer Speicherzelle selektiert ist, die mit der entsprechenden Wortleitung gekoppelt ist, wobei die erste Vorspannungsschaltungsbahn ein Lesespannungserzeugungsmittel (260) zum Erzeugen der Lesespannung umfasst.

7. Reihenselektor nach Anspruch 6, wobei die erste Vorspannungsschaltungsbahn ferner einen dritten Transistor (M3) zum Übertragen der Programmierspannung von dem genannten Programmierspannungserzeugungsmittel (215), wenn die genannte entsprechende Wortleitung zum selektiven Ausführen der Programmieropcration selektiert ist, und zum Übertragen der Lesespannung von dem genannten Lescspannungserzeugungsmittel (260) beinhaltet, wenn die genannte entsprechende Wortleitung zum selektiven Ausführen der Leseoperation selektiert ist, wobei der genannte dritte Transistor (M3) die Aufgabe hat, die Fähigkeit des Aushaltens von Spannungsdifferenzen, deren absolute Werte durch die Speisespannung des Halbleiterspeichers nach oben begrenzt werden, wenigstens über einen Steueranschluss und einen anderen Anschluss davon zu garantieren.

8. Reihenselektor nach Anspruch 6 oder 7 in Abhängigkeit von Anspruch 2, der ferner ein zweites Vorspannungsmittel (240, SW4, SW3) zum Steuern des dritten Transistors auf eine solche Weise, dass er leitend ist, wenn die entsprechende Wortleitung zum selektiven Ausführen der Programmoperation oder alternativ zum selektiven Ausführen der Leseoperation selektiert ist, und zum Steuern des dritten Transistors auf eine solche Weise beinhaltet, dass er während der Löschoperation nichtleitend ist.

9. Reihenselektor nach Anspruch 8, wobei die erste Schaltungsbahn einen vierten Transistor (M1) aufweist, der das zweite Vorspannungsmittel (240, SW4, SW3) mit einem Steueranschluss des dritten Transistors (M3) koppelt, wobei der vierte Transistor die Aufgabe hat, die Fähigkeit des Aushaltens von Spannungsdifferenzen, deren absolute Werte durch die Speisespannung nach oben begrenzt werden, wenigstens über einen Steueranschluss und einen anderen Anschluss davon zu garantieren.

10. Reihenselektor nach Anspruch 9, wobei das erste Vorspannungsmittel einen ersten Vorspannungsblock (220) aufweist, der die Aufgabe hat, ein den ersten Transistor steuerndes erstes Steuersignal (VB1) anzulegen, wobei das genannte erste Steuersignal auf einer ersten Selektiersteuerspannung (VB1s) ist, die bewirkt, dass der erste Transistor leitend ist, wenn die entsprechende Wortleitung zum selektiven Ausführen der Programmoperation selektiert ist, wobei das genannte erste Steuersignal auf einer ersten Unselektiersteuerspannung (VB1u) ist, wenn die entsprechende Wortleitung während der Programmoperation unselektiert ist, und während der Löschoperation auf einer ersten Löschsteuerspannung (VB1se) ist, wobei die erste Unselektieistcucrspannung und die erste Löschsteuerspannung bewirken, dass der erste Transistor nichtleitend ist.

11. Reihenselektor nach Anspruch 9 oder 10, wobei das erste Vorspannungsmittel einen zweiten Vorspannungsblock (225) aufweist mit der Aufgabe, ein den zweiten Transistor steuerndes zweites Steuersignal (VB2) anzulegen, wobei das genannte zweite Steuersignal auf einer zweiten Selektiersteuerspannung (VB2s) ist, die bewirkt, dass der zweite Transistor nichtleitend ist, wenn die entsprechende Wortleitung zum selektiven Ausführen der Programmoperation selektiert ist, wobei das genannte zweite Steuersignal auf einer zweiten Unselektiersteuerspannung (VB2u) ist, wenn die entsprechende Wortleitung während der Programmieroperation unselektiert ist, und während der Löschoperation auf einer zweiten Löschsteuerspannung (VB2se) ist, wobei die zweite Unselektiersteuerspannung und die zweite Löschsteuerspannung bewirken, dass der zweite Transistor leitend ist.

12. Reihenselektor nach Anspruch 11, wobei das Programmierspannungsemeugungsmittel und/oder der erste Vorspannungsblock und/oder der zweite Vorspannungsblock einen entsprechenden ersten Schaltungszweig (N4-N6; N22-N23; N32) und zweiten Schaltungszweig (N1-N3; N19-N21; N30-N31) aufweist/aufweisen, die mit einem entsprechenden Ausgangsschaltungsknoten (D60, D70, D80) verbunden sind, wobei der erste Schaltungszweig und der zweite Schaltungszweig die Aufgabe haben, an einem entsprechenden Eingangsanschluss davon ein entsprechendes erstes und zweites Betriebseingangssignal zu empfangen, wobei der genannte Ausgangsschaltungsknoten die Aufgabe hat, ein entsprechendes Ausgangssignal je nach dem ersten oder zweiten Betriebseingangssignal anzulegen, wobei der erste Schaltungszweig und der zweite Schaltungszweig eine entsprechende erste und zweite Mehrzahl von in Serie geschalteten Transistoren aufweisen, wobei eine entsprechende Anzahl von Transistoren in der genannten ersten Mehrzahl wenigstens gleich der kleinsten ganzen Zahl ist, die nicht kleiner ist als ein absoluter Wert eines Verhältnisses zwischen einer ersten Abfallspannung über den ersten Schaltungszweig, wenn der genannte erste Schaltungszweig nichtleitend ist, und der vorbestimmten Höchstspannung, wobei eine entsprechende Anzahl von Transistoren in der genannten zweiten Mehrzahl wenigstens gleich der kleinsten ganzen Zahl ist, die nicht kleiner ist als ein absoluter Wert eines Verhältnisses zwischen einer zweiten Abfallspannung über den zweiten Schaltungszweig, wenn der genannte zweite Schaltungszweig nichtleitend ist, und der vorbestimmten Höchstspannung.

13. Reihenselektor nach den Ansprüchen 6 bis 12, wobei die genannte Programmierspannung ein erstes Vielfaches der Speisespannung ist, die genannte Lesespannung ein zweites Vielfaches der Speisespannung ist, die genannte Löschspannung ein drittes Vielfaches der Speisespannung ist, die Programmierspannung und die Lesespannung ein erstes Vorzeichen haben, die Löschspannung ein zweites Vorzeichen hat, wobei das zweite Vorzeichen entgegengesetzt zum ersten Vorzeichen ist, wobei das erste Vielfache und/oder das zweite Vielfache und/oder das dritte Vielfache einen absoluten Wert hat/haben, der höher ist als der der Speisespannung.

14. Reihenselektor nach einem der Ansprüche 9 bis 13, wobei der erste, der zweite, der dritte und der vierte Transistor MOSFETs sind.

## Revendications

1. Un sélecteur de rangées pour une mémoire à semi-conducteurs comprenant une pluralité de cellules mémoire couplées à une pluralité correspondante de lignes de mots, le sélecteur de rangées comprenant, pour chaque ligne de mots :
- un premier trajet de circuit de polarisation (230; 215; 260) adapté de façon à polariser la ligne de mots correspondante vers une tension de programmation (POSV) lorsque ladite ligne de mots correspondante est sélectionnée de façon à exécuter de manière sélective une opération de programme sur au moins une cellule mémoire couplée à la ligne de mots correspondante, le premier trajet de circuit de polarisation comprenant un moyen de fourniture d'une tension de programmation (215 5) adapté de façon à fournir la tension de programmation (POSV),
- un deuxième trajet de circuit de polarisation (235) qui est adapté de façon à recevoir, d'un moyen de fourniture d'une tension d'inhibition de programme (250), une tension d'inhibition de programme (GND), et de façon à fournir à la ligne de mots correspondante ladite tension d'inhibition de programme (GND) lorsque la ligne de mots est désélectionnée au cours de l'opération de programme,
- un premier moyen de polarisation (220, 225) destiné à exciter le deuxième trajet de circuit de polarisation (235) afin de commander un état de conduction de celui-ci, où :
- ledit premier trajet de circuit de polarisation (230, 215, 260) comprend un premier transistor (M2) commandé de façon à être électriquement conducteur lorsque la ligne de mots correspondante est sélectionnée et à être électriquement non conducteur lorsque la ligne de mots correspondante est désélectionnée,
- ledit premier moyen de polarisation (220, 225) commande le deuxième trajet de circuit de polarisation de façon à être conducteur lorsque, au cours de l'opération de programme, la ligne de mots correspondante est désélectionnée,
- ledit deuxième trajet de circuit de polarisation (235) comprend une pluralité de transistors montés en série, le nombre de transistors dans ladite pluralité étant au moins égal à l'entier le plus petit non inférieur à une valeur absolue d'un rapport entre une tension égale à la différence entre la tension de programmation (POSV) et la tension d'inhibition de programme (GND) et une tension maximale prédéterminée,
**caractérisé en ce que** :
ladite tension maximale prédéterminée est la tension maximale qu'un transistor desdits transistors montés en série peut supporter avant de tomber en panne.

2. Le sélecteur de rangées selon la Revendication 1, où le deuxième trajet de circuit de polarisation est adapté de façon à recevoir d'un moyen de fourniture d'une tension d'effacement (250) une tension d'effacement (NEGV) et de façon à fournir à la ligne de mots correspondante ladite tension d'effacement au cours d'une opération d'effacement exécutée sur lesdites au moins deux lignes de mots de ladite pluralité, ledit premier moyen de polarisation commandant le deuxième trajet de circuit de polarisation de façon à être conducteur lors de l'exécution de l'opération d'effacement.

3. Le sélecteur de rangées selon la Revendication 1 ou 2, où chaque transistor de ladite pluralité est adapté de façon à garantir la capacité de supporter des différences de tension sur au moins une borne de commande et une autre borne de celui-ci qui sont limitées vers le haut en valeur absolue par une tension d'alimentation (Vdd) de la mémoire à semi-conducteurs.

4. Le sélecteur de rangées selon la Revendication 2 ou 3, où ladite pluralité de transistors comprend un deuxième transistor (M6) commandé par ledit premier moyen de polarisation de façon à être conducteur au cours de l'opération d'effacement et lorsque la ligne de mots correspondante est désélectionnée.

5. Le sélecteur de rangées selon la Revendication 2, 3 ou 4, où le premier transistor est adapté de façon à garantir la capacité de supporter, au moins sur une borne de commande et une autre borne de celui-ci, des différences de tension qui sont limitées vers le haut en valeur absolue par la tension d'alimentation.

6. Le sélecteur de rangées selon l'une quelconque des Revendications de 1 à 5, où le premier trajet de circuit de polarisation est adapté en outre de façon à polariser la ligne de mots correspondante sur une tension de lecture (Vcp1) lorsque ladite ligne de mots correspondante est sélectionnée de façon à exécuter de manière sélective une opération de lecture sur au moins une cellule mémoire couplée à la ligne de mots correspondante, le premier trajet de circuit de polarisation comprenant un moyen de fourniture d'une tension de lecture (260) adapté de façon à fournir la tension de lecture.

7. Le sélecteur de rangées selon la Revendication 6, où le premier trajet de circuit de polarisation comprend en outre un troisième transistor (M3) adapté de façon à transmettre la tension de programmation dudit moyen de fourniture d'une tension de programmation (215) lorsque ladite ligne de mots correspondante est sélectionnée de façon à exécuter de manière sélective l'opération de programme et de façon à transmettre la tension de lecture dudit moyen de fourniture d'une tension de lecture (260) lorsque ladite ligne de mots correspondante est sélectionnée de façon à exécuter de manière sélective l'opération de lecture, ledit troisième transistor (M3) étant adapté de façon à garantir la capacité de supporter au moins sur une borne de commande et une autre borne de celui-ci des différences de tension qui sont limitées vers le haut en valeur absolue par la tension d'alimentation de la mémoire à semi-conducteurs.

8. Le sélecteur de rangées selon la Revendication 6 ou 7 dépendante de la Revendication 2, comprenant en outre un deuxième moyen de polarisation (240, SW4, SW3) destiné à commander le troisième transistor de façon à être conducteur lorsque la ligne de mots correspondante est sélectionnée de façon à exécuter de manière sélective l'opération de programme ou, selon une variante, de façon à exécuter de manière sélective l'opération de lecture, et de façon à commander le troisième transistor à être non-conducteur au cours de l'opération d'effacement.

9. Le sélecteur de rangées selon la Revendication 8, où le premier trajet de circuit comprend un quatrième transistor (M1) reliant le deuxième moyen de polarisation (240, SW4, SW3) à une borne de commande du troisième transistor (M3), le quatrième transistor étant adapté de façon à garantir la capacité de supporter, au moins sur une borne de commande et une autre borne de celui-ci, des différences de tension qui sont limitées vers le haut en valeur absolue par la tension d'alimentation.

10. Le sélecteur de rangées selon la Revendication 9, où le premier moyen de polarisation comprend un premier bloc de polarisation (220) qui est adapté de façon à fournir un premier signal de commande (VB1) commandant le premier transistor, ledit premier signal de commande étant réglé sur une première tension de commande de sélection (VB1s) adaptée de façon à amener le premier transistor à être conducteur lorsque la ligne de mots correspondante est sélectionnée de façon à exécuter de manière sélective l'opération de programme, ledit premier signal de commande étant réglé sur une première tension de commande de désélection (VB1u) lorsque la ligne de mots correspondante est désélectionnée au cours de l'opération de programme et sur une première tension de commande d'effacement (VB1se) au cours de l'opération d'effacement, la première tension de commande de désélection et la première tension de commande d'effacement étant adaptées de façon à amener le premier transistor à être non-conducteur.

11. Le sélecteur de rangées selon la Revendication 9 ou 10, où le premier moyen de polarisation comprend un deuxième bloc de polarisation (225) qui est adapté de façon à fournir un deuxième signal de commande (VB2) commandant le deuxième transistor, ledit deuxième signal de commande étant réglé sur une deuxième tension de commande de sélection (VB2s) adaptée de façon à amener le deuxième transistor à être non-conducteur lorsque la ligne de mots correspondante est sélectionnée de façon à exécuter de manière sélective l'opération de programme, ledit deuxième signal de commande étant réglé sur une deuxième tension de commande de désélection (VB2u) lorsque la ligne de mots correspondante est désélectionnée au cours de l'opération de programme et sur une deuxième tension de commande d'effacement (VB2se) au cours de l'opération d'effacement, la deuxième tension de commande de désélection et la deuxième tension de commande d'effacement étant adaptées de façon à amener le deuxième transistor à être conducteur.

12. Le sélecteur de rangées selon la Revendication 11, où au moins un des éléments parmi le moyen de fourniture d'une tension de programmation, le premier bloc de polarisation et le deuxième bloc de polarisation comprend une première branche de circuit correspondante (N4-N6; N22-N23; N32) et une deuxième branche de circuit (N 1-N3; N 19-N21; N30-N31) qui sont reliées à un noeud de circuit de sortie correspondant (D60, D70, D80), la première branche de circuit et la deuxième branche de circuit étant adaptées de façon à recevoir sur une borne d'entrée correspondante de celles-ci un premier et un deuxième signal d'entrée opérationnel correspondants, ledit noeud de circuit de sortie étant adapté de façon à fournir un signal de sortie correspondant en fonction du premier ou du deuxième signal d'entrée opérationnel, la première branche de circuit et la deuxième branche de circuit comprenant une première et une deuxième pluralités correspondantes de transistors montés en série, un nombre correspondant de transistors dans ladite première pluralité étant au moins égal à l'entier le plus petit non inférieur à une valeur absolue d'un rapport entre une première tension de chute sur la première branche de circuit lorsque ladite première branche de circuit est non conductrice et la tension maximale prédéterminée, un nombre correspondant de transistors dans ladite deuxième pluralité étant au moins égal à l'entier le plus petit non inférieur à une valeur absolue d'un rapport entre une deuxième tension de chute sur la deuxième branche de circuit lorsque ladite deuxième branche de circuit est non conductrice et la tension maximale prédéterminée.

13. Le sélecteur de rangées selon la Revendication 6 à 12, où ladite tension de programmation est un premier multiple de la tension d'alimentation, ladite tension de lecture est un deuxième multiple de la tension d'alimentation, ladite tension d'effacement est un troisième multiple de la tension d'alimentation, la tension de programmation et la tension de lecture possédant un premier signe, la tension d'effacement possédant un deuxième signe, le deuxième signe étant opposé au premier signe, au moins un multiple parmi le premier multiple, le deuxième multiple et le troisième multiple étant supérieur en valeur absolue à la tension d'alimentation.

14. Le sélecteur de rangées selon l'une quelconque des Revendications 9 à 13, où le premier, le deuxième, le troisième et le quatrième transistors sont des transistors MOSFET.
